(19) Europäisches Patentamt / European Patent Office / Office européen des brevets

(11) **EP 4 137 063 B1**

(12) **EUROPEAN PATENT SPECIFICATION**

(45) Date of publication and mention
of the grant of the patent:
**11.12.2024 Bulletin 2024/50**

(21) Application number: **21787968.3**

(22) Date of filing: **15.04.2021**

(51) International Patent Classification (IPC):
*A61B 8/12* (2006.01)     *A61B 8/08* (2006.01)
*A61B 5/1459* (2006.01)     *G01N 33/487* (2006.01)

(52) Cooperative Patent Classification (CPC):
**A61B 8/12; A61B 5/1459; A61B 8/085;
A61B 8/4444; A61B 8/4494; G01N 33/487**

(86) International application number:
**PCT/JP2021/015576**

(87) International publication number:
**WO 2021/210642 (21.10.2021 Gazette 2021/42)**

(54) **HEMOGLOBIN CONCENTRATION MEASURING SYSTEM AND HEMOGLOBIN CONCENTRATION MEASURING METHOD**

HÄMOGLOBINKONZENTRATIONSMESSSYSTEM UND HÄMOGLOBINKONZENTRATIONSMESSVERFAHREN

SYSTÈME DE MESURE DE CONCENTRATION D'HÉMOGLOBINE ET PROCÉDÉ DE MESURE DE CONCENTRATION D'HÉMOGLOBINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.04.2020 PCT/JP2020/016638**

(43) Date of publication of application:
**22.02.2023 Bulletin 2023/08**

(73) Proprietor: **Cellspect Co., Ltd.
Morioka-shi, Iwate 020-0857 (JP)**

(72) Inventors:
• **SAKAI Hironori**
  **Morioka-city, Iwate 0200857 (JP)**
• **IWABUCHI Takuya**
  **Morioka-city, Iwate 0200857 (JP)**

(74) Representative: **Hasegawa, Kan
Patentanwaltskanzlei Hasegawa
Untere Hauptstraße 56
85354 Freising (DE)**

(56) References cited:
EP-A1- 3 124 977     WO-A1-2014/011403
WO-A1-2015/147284     JP-A- 2000 516 112
JP-A- 2010 029 399     JP-A- 2010 516 305
JP-A- 2013 544 551     JP-A- 2019 213 570
US-A1- 2005 065 436     US-A1- 2010 256 461
US-A1- 2015 173 626     US-A1- 2018 214 119

• ANONYMOUS: "Development of Optical Transvaginal Probe that can Evaluate the Malignant transformation of Endometriosis in a Minimally Invasive Manner (Report)", 1 October 2018 (2018-10-01), pages 1 - 16, XP093132411, Retrieved from the Internet <URL:https://www.chusho.meti.go.jp/keiei/sapoin/portal/seika/28fy.htm> [retrieved on 20240216]
• ANONYMOUS: "Development of Optical Transvaginal Probe that can Evaluate the Malignant transformation of Endometriosis in a Minimally Invasive Manner (Summary)", 1 October 2018 (2018-10-01), pages 1 - 1, XP093132413, Retrieved from the Internet <URL:https://www.chusho.meti.go.jp/keiei/sapoin/portal/seika/28fy.htm> [retrieved on 20240216]
• TAKUYA IWABUCHI ET AL: "Cyst fluid hemoglobin species in endometriosis and its malignant transformation: The role of metallobiology", ONCOLOGY LETTERS, 29 March 2016 (2016-03-29), GR, XP055399582, ISSN: 1792-1074, DOI: 10.3892/ol.2016.4383

EP 4 137 063 B1

- "Development of transvaginal optical probe that can evaluate the malignization of endometriosis with minimally invasive", STRATEGIC FUNDAMENTAL TECHNOLOGY ENHANCEMENT AND COOPERATION SUPPORT PROJECT 2017, STRATEGIC FUNDAMENTAL TECHNOLOGY IMPROVEMENT SUPPORT OPERATION, REPORT OF RESEARCH AND DEVELOPMENT RESULTS, 24 June 2020 (2020-06-24), pages 1 - 14
- YAMADA, YUKI: "O-043: New diagnostic method for malignant transformation of the chocolate cyst using near-infrared radiation", PROGRAMS AND ABSTRACTS OF THE 39TH ACADEMIC LECTURE CONFERENCE OF THE JAPAN SOCIETY OF ENDOMETRIOSIS; JANUARY 27-28, 2018, vol. 39, 5 January 2018 (2018-01-05), JP, pages 139, XP009541017
- KOBAYASHI, H. ET AL.: "Modern approaches to noninvasive diagnosis of malignant transformation of endometriosis", ONCOLOGY LETTERS, vol. 17, no. 1, January 2019 (2019-01-01), pages 1196 - 1202, XP055867755, [retrieved on 20181116], DOI: 10.3892/ol.2018.9721
- NIIRO, EMIKO: "O5-4: Development of ultrasonic equipment aiming at early detection of malignization in ovarian endometriosis", PROGRAMS AND ABSTRACTS OF THE 41ST ACADEMIC LECTURE CONFERENCE OF THE JAPAN SOCIETY OF ENDOMETRIOSIS; JANUARY 18-19, 2020, vol. 41, 7 January 2020 (2020-01-07), JP, pages 104, XP009541016
- "Main technology: Measurement and instrumentation technique", 24 June 2020, WARP, JP, article CELLSPECT (IWATE-KEN): "Development of transvaginal optical probe that can evaluate the malignization of endometriosis with minimally invasive", pages: 2, XP009542604
- ANONYMOUS: "Small and Medium Enterprise Agency: Research and Development Results Report", 6 January 2020 (2020-01-06), JP, pages 1 - 8, XP009540862, Retrieved from the Internet <URL:https://warp.ndl.go.jp/info:ndljp/pid/11424571/www.chusho.meti.go.jp/keiei/sapoin/portal/seika/28fy.htm> [retrieved on 20200624]

**Description**

TECHNICAL FIELD

The present invention relates to a technique to measure the hemoglobin concentration in the cyst fluid of an endometriotic ovarian cyst in vivo.

BACKGROUND ART

Endometriosis is a gynecological disease that occurs in about 1 in 10 women. An endometriotic ovarian cyst, which is a kind of endometriosis, is a benign ovarian cyst, also known as a "chocolate cyst," in which blood bled from the endometriosis developed in the ovary accumulates and forms a cyst.

It is reported that approximately 1% of endometriotic ovarian cysts cancerate into ovarian cancer. Ovarian cancer arising from endometriosis is collectively referred to as endometriosis associated ovarian cancer (EAOC). From an epidemiological point of view, oophorectomy is currently recommended for patients aged 44 years or over, with a cyst diameter of 84 mm or larger, and with a rapid increase in cysts in a short period of time, because the risk of carcinogenesis is considered to be high. So far, however, only in the order of 1% of cases have actually been determined as malignant by postoperative pathologist diagnosis. This means that 99% of cases have undergone oophorectomy despite such cases being benign.

As a technique related to determining the degree of malignancy of endometriotic ovarian cysts, NPTL 1 discloses the correlation between the degree of malignancy of endometriotic ovarian cysts and biomarker concentration.

In addition, PTL 1 discloses a method for determining the possibility of an endometriotic ovarian cyst being cancerous to be performed in a diagnostic device equipped with: an iron concentration measurement part which measures an iron concentration in a collected cyst fluid of a endometriotic ovarian cyst; and a determination part which includes a presence ratio measuring part for measuring the presence ratio of met-heme/oxy-heme in the cyst fluid of the endometriotic ovarian cyst and determines the possibility of the cyst being cancerous. PTL 1 illustrates a Triton-MeOH assay chromogenic method and a High-performance liquid chromatography method as examples of methods for measuring the total heme iron concentration in the cystic fluid. In addition, near-infrared spectroscopy, nuclear magnetic resonance spectroscopy (MRS), activation analysis, and X-ray fluorescence analysis are illustrated as examples of methods for measuring the iron concentration in the cystic fluid with the cystic fluid being contained in the endometriotic ovarian cyst. Further, PTL 1 discloses an iron concentration measurement part, which includes a probe provided with a light emitting part for emitting near-infrared light and a light receiving part for receiving near-infrared light.

In addition, PTL 2 discloses an analytical method for blood using near-infrared spectroscopy, in which: light is applied to the blood in a translucent blood collection tube or bag from the outside through the blood collection tube or bag; scattered reflected light, scattered transmitted light, or transmitted and reflected light from the blood within the blood collection tube or bag is detected using an optical sensor to measure a near infrared absorption spectrum of the blood; and the measurements are substituted into a calibration curve, which has been made in advance from a spectrum measured using the same method, to determine chemical components or physicochemical characteristics of the blood. In this analytical method for blood, near-infrared light with wavelengths between 700 nm and 1100 nm is applied to the blood within the above-described translucent blood collection tube or bag.

PTL 3 discloses a diagnostic probe that acquires data for diagnosing endometriotic ovarian cysts.

NPTL 2 discloses that near-infrared light is applied to a simulated cyst containing a hemoglobin solution of a known concentration, the hemoglobin concentration is calculated based on the optical spectrum of the reflected light, and a multiple regression formula is constructed, which uses multiple wavelengths by multivariate analysis.

PTL 4 describes a diagnostic device and corresponding method for diagnosing a likelihood of canceration of endometriosis ovarian cyst. An iron concentration in the cystic fluid is determined by determining the quantity of hemoglobin by near-infrared spectroscopy. A ratio of met-heme/oxy-heme in the cyst fluid is measured by measuring the sensitivity of two wavelengths (absorbance or optical density) to which met-heme and oxy-heme attribute respectively, and calculating a sensitivity ratio of the sensitivities obtained. An image forming by ultrasound is carried out in order to confirm a position of the endometriosis ovarian cysts of interest for diagnosing tumor in the image.

PRIOR ART DOCUMENTS

PATENT LITERATURE

PTL 1: JP2018-163174 A

PTL 2: JP2002-122537 A

PTL 3: JP6657438 B

PTL 4: EP 3 124 977 A1

NON-PATENT LITERATURE

NPTL 1: C. Yoshimoto et. al., "Cyst fluid iron-related compounds as useful markers to distinguish malignant transformation from benign endometriotic cysts," Cancer Biomarkers, vol. 15 (2015), pp. 493-499

NPTL 2: "Development of Optical Transvaginal Probe that can Evaluate the Malignant transformation of Endometriosis in a Minimally Invasive Manner)," Report on Research and Development Result- Strategic Fundamental Technology Advancement Support Project - Strategic Fundamental Technology Advancement/Cooperation Support Project in FY2017, [URL: https://www.chusho.meti.go.jp/keiei/sapoin/portal/seika/28fy.htm], posted in October 2018

PROBLEMS RELATED TO THE BACKGROUND ART

The degree of malignancy (the possibility of carcinogenesis) of endometriotic ovarian cysts has traditionally been estimated based on the morphological assessment of ovarian cysts using ultrasound diagnosis apparatuses, computed tomography (CT), and nuclear magnetic resonance imaging (MRI). However, according to the fact that cancerous endometriotic ovarian cysts account for 1% of pathological diagnosis after oophorectomy, it is in fact difficult to distinguish between benign endometriotic ovarian cysts and cancerous endometriotic ovarian cysts based on this morphological assessment.

As a method to recognize cancerous areas, contrast-enhanced MRI examinations are present, in which a patient is given a contrast agent and an MRI is performed. However, this method is burdensome to the patient, and there is also a risk of contrast agent side effects. There is also a problem that it cannot be performed in regular outpatient care. Therefore, it is not possible to perform contrast-enhanced MRI examinations frequently, and depending on the frequency of examinations, cancerous cases may be missed.

Thus, there is currently no simple and non-invasive method for determining the degree of malignancy of endometriotic ovarian cysts with high reliability. Therefore, it is currently difficult to examine patients diagnosed with endometriotic ovarian cyst regularly in outpatient care and to deliver treatment according to the degree of progression of the disease. As a result, when diagnosed with endometriotic ovarian cysts, many patients undergo surgical procedures of the endometriotic ovarian cyst by removing the entire ovary in consideration of the possibility of the cyst developing into cancer in the future, despite it not being cancerous at the current time.

To deal with this problem, according to PTL 1, the degree of malignancy of endometriotic ovarian cyst can be determined more easily and with higher reliability than before by using hemoglobin in the cyst fluid as a biomarker. In addition, if the hemoglobin concentration in the cyst fluid can be measured in situ (in vivo) and non-invasively, the burden on the patient may be reduced.

The concentration of a certain component in a liquid can be measured in a contactless manner by spectroscopy utilizing the optical spectrum of reflected light reflected from the liquid or transmitted light transmitted through the liquid (see, for example, PTL 2). However, the cyst fluid is retained, in vivo, in amorphous and inhomogeneous tissues, such as stromal cells and visceral fat. Therefore, even if light is applied to the ovarian cyst in vivo in order to measure the hemoglobin concentration in the cystic fluid non-invasively, the noise in the optical spectra obtained thereby may become extremely large due to the presence of tissues, such as stromal cells and visceral fat. Measuring hemoglobin concentration by spectroscopy based on such optical spectra fails to provide sufficient measurement accuracy when compared with collecting the cyst fluid from the ovarian cyst and measuring the concentration in vitro.

In contrast, in NPTL 2, the above-described effects of the tissues, such as stromal cells and visceral fat, can be reduced even if the hemoglobin concentration in the cyst fluid is measured in vivo by constructing a multiple regression formula based on the measurement values obtained in the experiment in which near-infrared light is applied to the simulated cyst.

However, in order to measure the hemoglobin concentration in the cyst fluid in vivo, it is necessary to reliably apply light to the cyst fluid to be measured. In this regard, PTL 3 and NPLT 2 describe that the location of the ovarian cyst is confirmed by using ultrasound images, but the detailed structure is not articulated, and it is therefore difficult to reliably apply light to a measurement target and stably perform the measurements. PTL 4 discloses a transvaginal diagnostic device comprising a probe for measuring the iron concentration and a probe of an ultrasound image forming unit, wherein the two probes may be integrated in a single insertion member.

PROBLEMS TO BE SOLVED BY THE INVENTION

The present invention has been made in view of the above-described problems, and an object thereof is to provide a hemoglobin concentration measuring system and a hemoglobin concentration measuring method, which are capable of measuring hemoglobin concentration in the cystic fluid, which is retained in the ovarian cyst in the living body, in a non-invasive and stable manner.

SUMMARY OF THE INVENTION

In order to solve the above-described problems, the hemoglobin concentration measuring system, which is an aspect of the present invention, is provided by the features of claim 1.

In the above-described hemoglobin concentration measuring system, the ultrasound transmitting/receiving part may transmit the ultrasound so as to scan the living tissue in a convex manner, and the light irradiation part may be provided such that the light irradiation part is capable of emitting the light in a direction parallel to an ultrasound transmission direction at the center of the convex-shaped scanning plane.

In the above-described hemoglobin concentration measuring system, the light irradiation part and the light receiving part may be arranged opposite each other with the ultrasound transmitting/receiving part sandwiched therebetween.

In the above-described hemoglobin concentration measuring system, the light irradiation part and the light receiving part may be arranged such that a line connecting an end surface of the light irradiation part and an end surface of the light receiving part is orthogonal to a scanning direction of the ultrasound transmitted from the ultrasound transmitting/receiving part.

In the above-described hemoglobin concentration measuring system, the center-to-center distance between the end surface of the light irradiation part and the end surface of the light receiving part may be between 10 mm and 31 mm, inclusive.

In the above-described hemoglobin concentration measuring system, the ultrasound transmitting/receiving part may be provided on a transvaginal ultrasound probe, the light irradiation part and the light receiving part may be attached to a holder to be placed over an insertion part, which is to be inserted into the vagina, of the ultrasound probe, and the light irradiation part and the light receiving part, along with the holder, may be detachable from the ultrasound probe.

In the above-described hemoglobin concentration measuring system, the concentration calculation part acquires absorbance measurement values at the plurality of specific wavelengths, and calculate the hemoglobin concentration by substituting the absorbance measurement values into a pre-acquired predetermined formula that represents the relationship between absorbance at the plurality of specific wavelengths and hemoglobin concentration.

In the above-described hemoglobin concentration measuring system, the components of the plurality of wavelengths includes, at least, components of a wavelength in a visible light region.

In the above-described hemoglobin concentration measuring system, the plurality of specific wavelengths may include, at least, two or more wavelengths selected from: 580 nm, 590 nm, 640 nm, 680 nm, and 762 nm.

The above-described hemoglobin concentration measuring system may further comprise a determination part that determines under which classifications of degree of malignancy of an endometriotic ovarian cyst pre-associated with hemoglobin concentration the hemoglobin concentration calculated by the concentration calculation part falls.

The hemoglobin concentration measuring method, which is another aspect of the present invention, is provided by the features of claim 10.

According to the present invention, since light is emitted in a predetermined direction with respect to the ultrasound scanning plane, the light can be reliably applied to the living tissue by referring to the ultrasonic image, and the reflected light or transmitted light thereof can be received. Accordingly, the hemoglobin concentration in the cyst fluid retained in the ovarian cyst in the living body can be measured in a non-invasive and stable manner.

BRIEF DESCRIPTION OF THE DRAWINGS

[FIG. 1] FIG. 1 is a table showing the definite diagnoses after oophorectomy and hemoglobin concentration.
[FIG. 2] FIG. 2 is a graph representing hemoglobin concentration and cutoff values belonging to non-cancer and cancer groups.
[FIG. 3] FIG. 3 is a flowchart showing a method of measuring biomarker concentration according to a first embodiment of the present invention.
[FIG. 4] FIG. 4 is a flowchart showing the method of determining the formula used in the concentration calculation step shown in FIG. 3.

[FIG. 5] FIG. 5 is a block diagram illustrating a schematic configuration of a hemoglobin concentration measuring system according to a first embodiment of the present invention.

[FIG. 6] FIG. 6 is a plan view schematically showing a measuring optical system used in Example 1-1.

[FIG. 7] FIG. 7 is a graph showing the second derivative of the optical spectra in Example 1-1.

[FIG. 8] FIG. 8 is a graph showing the correlation between the directly-measured hemoglobin concentration and the calculated hemoglobin concentration (using all wavelengths) in Example 1-1.

[FIG. 9] FIG. 9 is a graph showing the correlation between the directly-measured hemoglobin concentration and the calculated hemoglobin concentration (using 12 wavelengths) in Example 1-2.

[FIG. 10] FIG. 10 is a graph showing the correlation between the directly-measured hemoglobin concentration and the calculated hemoglobin concentration (using two wavelengths) in Example 1-3.

[FIG. 11] FIG. 11 is a table showing the wavelengths used in formation of the regression formula and correlation coefficients in Example 1-4.

[FIG. 12] FIG. 12 is a graph showing the second derivative of the optical spectra in Example 2-1.

[FIG. 13] FIG. 13 is a graph showing the correlation between the directly-measured hemoglobin concentration and the calculated hemoglobin concentration (using all wavelengths) in Example 2-1.

[FIG. 14] FIG. 14 is a graph showing the correlation between the directly-measured hemoglobin concentration and the calculated hemoglobin concentration (using two wavelengths) in Example 2-2.

[FIG. 15] FIG. 15 is a plan view schematically showing a tip portion of a probe used in Example 3.

[FIG. 16] FIG. 16 is a graph showing the correlation between the directly-measured hemoglobin concentration and the calculated hemoglobin concentration in Example 3.

[FIG. 17] FIG. 17 is a side view schematically showing a transvaginal probe according to a second embodiment of the present invention.

[FIG. 18] FIG. 18 is a top view of the transvaginal probe shown in FIG. 17.

[FIG. 19] FIG. 19 is an enlarged view seen from arrow X in FIG. 17.

[FIG. 20] FIG. 20 is a schematic diagram for explaining the relationship between an ultrasound image and the direction of light application.

[FIG. 21] FIG. 21 is a graph showing the correlation between the directly-measured hemoglobin concentration and the calculated hemoglobin concentrations(using two wavelengths) in Example 4.

## EMBODIMENTS OF THE INVENTION

Hereinafter, a hemoglobin concentration measuring system and a hemoglobin concentration measuring method according to embodiments of the present invention will be described with reference to the drawings. It should be noted that the present invention is not limited by these embodiments. In the description of each drawing, the same parts are denoted by the same reference numbers.

The drawings referred to in the following description are merely schematic representations of shape, size, and positional relationship to the extent that the subject matter of the present invention may be understood. In other words, the present invention is not limited only to the shapes, sizes, and positional relationships illustrated in the respective figures. In addition, the drawings may also include, among themselves, parts having different dimensional relationships and ratios from each other. In this specification, unless otherwise specified, the numerical range "A to B" refers to "equal to or greater than A, and equal to or less than B."

In the following embodiments, hemoglobin in the cystic fluid retained in an endometriotic ovarian cyst is described as an example of a biomarker, which is a target to be measured for concentration. However, the method of measuring biomarker concentration according to the present invention is not limited to the hemoglobin concentration in the cystic fluid in the ovarian cyst, and it may be applied to cases in which the concentration of various components in the fluid retained in the living tissue is to be measured while the fluid is retained in the living tissue.

The cyst fluid of an endometriotic ovarian cyst is the fluid that has accumulated inside the endometriotic ovarian cyst. Hemoglobin in the cystic fluid can be used as a biomarker when determining the possibility (i.e., the degree of malignancy) of an endometriotic ovarian cyst being cancerous. There are no particular limitations on subjects as long as they are organisms that may develop endometriotic ovarian cysts, and this includes mammals in general.

The degree of malignancy of an endometriotic ovarian cyst may be determined based on the knowledge that the concentration of hemoglobin contained in the cyst fluid of a cancerous endometriotic ovarian cyst is significantly lower than the hemoglobin concentration in the cyst fluid of a benign endometriotic ovarian cyst.

First, the association between the hemoglobin concentration in the cystic fluid and the degree of malignancy of the endometriotic ovarian cyst will be described based on a statistical analysis of hemoglobin concentration measurements in clinical analytes.

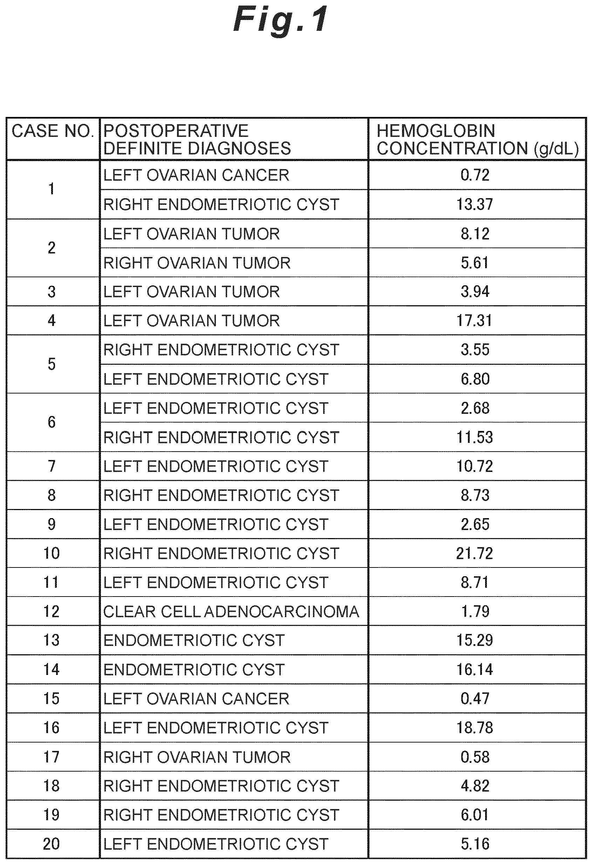

When the inventors of the present application measured the hemoglobin concentration in the cyst fluids provided by patients who had undergone oophorectomy, it became clear that there was an association between the hemoglobin concentration in the cyst fluid and the degree of malignancy of the cyst. FIG. 1 is a table showing the postoperative definite diagnoses and hemoglobin concentration in some cases. The hemoglobin concentration measurement was performed by using a metallo assay LS heme assay kit (manufactured by Metallogenics Co., Ltd.) and measuring electron absorption spectra with a microplate reader (manufactured by Corona Electric Co., Ltd., type name: SH-1200).

According to postoperative pathologist diagnosis, out of 117 cases, 88 cases were diagnosed to be in the non-cancer group and 29 cases were diagnosed to be in the cancer group. The following table shows the mean, standard deviation, range, and significance probability (p-value) of the measurements.

[Table 1]

|  | Non-cancer group | Cancer group | p-value |
| --- | --- | --- | --- |
| Hemoglobin concentration (g/dL) | 5.7 +/- 12.9 (0.4-110.1) | 0.9 +/- 1.4 (0.1-5.4) | < 0.001 |
| Mean +/- Standard Deviation |  |  |  |

As shown in Table 1, the mean hemoglobin concentration in the non-cancer group is 5.7 g/dL and the mean hemoglobin concentration in the cancer group is 0.9 g/dL. Thus, the hemoglobin concentration in the non-cancer group is clearly higher than the hemoglobin concentration in the cancer group. In addition, since the p-value is less than 0.001, it is clear that there is a correlation between the hemoglobin concentration and the degree of malignancy.

As a result of a ROC curve analysis on these results, a cutoff value of 2.0 g/dL was calculated for the cancer and non-cancer groups. The sensitivity and specificity at this time were 95.4% and 81.1%, respectively. In addition, the positive predictive value and negative predictive value when this cutoff value was used were 95.4% and 85.7%, respectively.

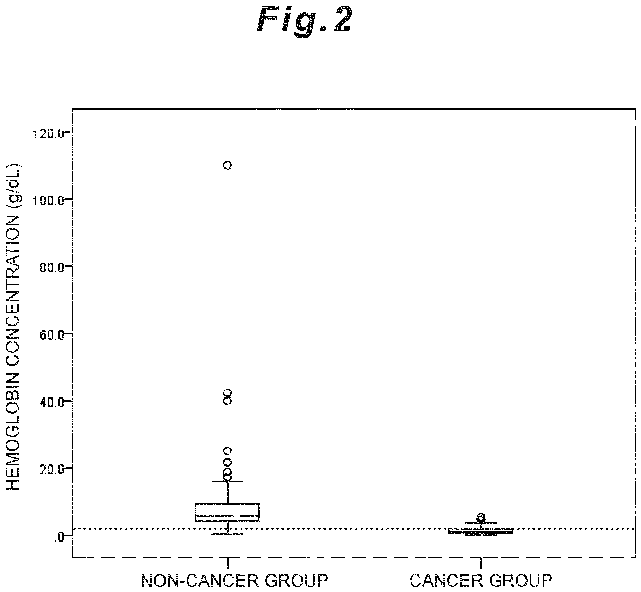

FIG. 2 is a graph showing the results of a nonparametric analysis (Mann-Whitney U-test) of the difference in the mean hemoglobin concentration in the cystic fluid in the non-cancer and cancer groups. In FIG. 2, the cutoff value is shown by a dashed line.

Based on the above analysis, it is possible to determine the possibility of carcinogenesis with a probability of 85.7% when the hemoglobin concentration in the analyte is between 0.0 and 2.0 g/dL.

The relationship between the numerical range of hemoglobin concentration and the determination of the degree of malignancy of endometriotic ovarian cysts is also described in C. Yoshimoto et al., "Cyst fluid iron-related compounds as useful markers to distinguish malignant transformation from benign endometriotic cysts" (Cancer Biomarkers, vol. 15 (2015), P. 493-499). In this literature, the cutoff value is 72.7 mg/dL of the heme iron concentration (see page 497), which corresponds to a hemoglobin concentration of 2.0 g/dL.

(First Embodiment)

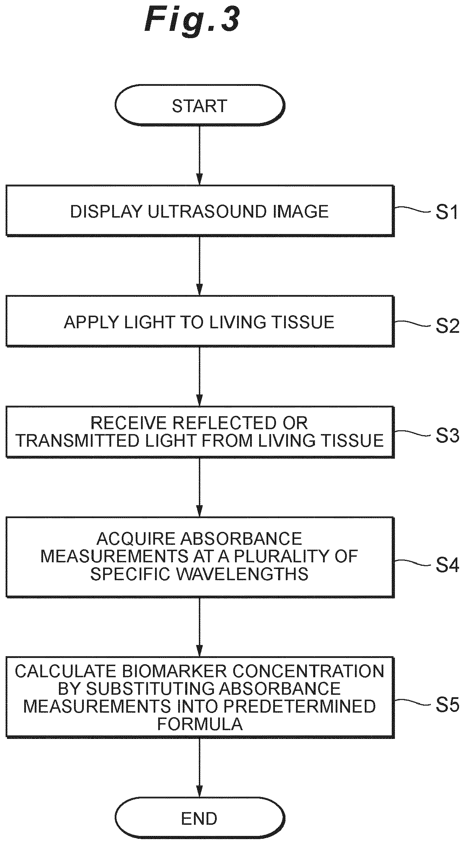

FIG. 3 is a flowchart showing a measuring method according to a first embodiment of the present invention. The measuring method according to the present embodiment is a method for measuring the biomarker concentration in the fluid retained in the living tissue in a non-invasive manner in situ (in vivo) while the fluid is retained in the living tissue. As an example, the case where the hemoglobin concentration in the cyst fluid retained in an endometriotic ovarian cyst of a subject is described below.

First, an ultrasound image containing an image of a target living tissue is displayed using an ultrasound observation device and the location of such living tissue is confirmed (ultrasound image display step S1). For example, when measuring the hemoglobin concentration in the cystic fluid retained in the ovarian cyst, a transvaginal ultrasound probe may be used to display an ultrasound image containing an image of the ovarian cyst.

Then, light in the wavelength region ranging from visible light to near-infrared light is applied to the living tissue retaining the fluid (light irradiation step S2). The light applied is not particularly limited if it is light containing components of a plurality of specific wavelengths (described below), and it may be visible light only, near-infrared light only, or light containing both. In accordance with the invention the components of the plurality of wavelengths include, at least, components of a wavelength in a visible light region. The plurality of specific wavelengths are determined according to a formula used in the concentration calculation step S5 described below. For example, when measuring the hemoglobin concentration in the cystic fluid in an ovarian cyst, the light applied is preferably light containing components of at least two wavelengths selected from among 580 nm, 590 nm, 640 nm, 680 nm, 762 nm, 876 nm, 900 nm, 932 nm, 958 nm, 968 nm, 978 nm, and 1095 nm. A light source that generates the light applied is not particularly limited if the light source can apply light to the living body in a non-invasive manner. For example, a light source combining multiple LEDs with different peak wavelengths may be used, or a halogen lamp may be used. In addition, the method for applying light is not particularly limited if it is non-invasive with respect to the living body, and the light may be applied transvaginally or transabdominally. For example, an optical fiber may be connected to a light source and the end of the optical fiber may be made to contact with the living body, and thereby, light may be applied to a local area of the living body.

Next, reflected light reflected by the living tissue or transmitted light transmitted through the living tissue is received (light receiving step S3). The light-receiving means for the reflected light or transmitted light is not particularly limited if it is non-invasive and capable of detecting light intensity at the above-described plurality of specific wavelengths. For example, a combination of multiple spectroscopic sensors that are sensitive only to specific wavelengths may be used, or a sensor that is capable of detecting wavelengths of a wideband may be used as the light-receiving means. In addition, optical fibers may be connected to these sensors and the ends of the optical fibers may be made to contact with the living body, and thereby, the light from a local area of the living body may be received.

In the light irradiation step S2 and the light receiving step S3, for example, light irradiation means and light receiving means may be provided on a transvaginal probe, and light may be applied from within the vagina and reflected light may be received within the vagina. In addition, light application means and light receiving means may be provided on a transabdominal probe, and light may be applied from the abdominal surface and reflected light may be received at the abdominal surface. Alternatively, light application means and light receiving means may be provided on each of a transabdominal probe and a transvaginal probe, and light may be applied from the abdominal surface and the transmitted light may be received within the vagina, and vice versa.

Then, based on the light received in the light receiving step S3, absorbance measurement values at the plurality of specific wavelengths are acquired (absorbance acquisition step S4). As described above, the plurality of specific wavelengths are determined according to a formula used in the concentration calculation step S5 described below. The absorbance $A(\lambda)$ at a wavelength $(\lambda)$ may be calculated according to the following formula (1) by using the intensity of the light applied $I_0$ and the intensity of the reflected light or transmitted light $I_1$ based on the Beer-Lambert law:

$$A(\lambda) = -\log_{10}(I_1/I_0) \qquad \ldots (1)$$

The biomarker concentration is then calculated by substituting the absorbance measurement values acquired in the absorbance acquisition step S4 into a pre-acquired predetermined formula that represents the relationship between absorbance at the plurality of specific wavelengths and biomarker concentration (concentration calculation step S5). The biomarker concentration C is represented by, for example, the following formula (2) that uses the absorbance $A(\lambda_n)$ (n = 1 to N, N ≥ 2) at a specific wavelength $\lambda_n$ as a variable. In formula (2), the coefficient $a_n$ is pre-set. The sign b is a constant.

$$C = a_1 A(\lambda_1) + a_2 A(\lambda_2) + \ldots + a_N A(\lambda_N) + b \qquad \ldots (2)$$

In this way, the biomarker concentration C can be obtained in a non-invasive manner.

Next, the method of determining the formula used in the concentration calculation step S5 will be described.

As described above, the degree of malignancy of an endometriotic ovarian cyst can be determined with high reliability by measuring the hemoglobin concentration in the cyst fluid. However, because the cyst fluid is retained in amorphous and inhomogeneous living tissues, such as stromal cells and visceral fat, when light is applied to the cyst fluid through the living tissue (i.e., without invading the living tissue), the noise becomes extremely large in the optical spectrum of the reflected light or transmitted light. Therefore, even if the hemoglobin concentration is measured by general spectroscopy using the Beer-Lambert law based on such noisy optical spectrum, sufficient measurement accuracy cannot be obtained. In the first place, it is also difficult to determine the optical path length in the living body. In other words, quantitative analysis by spectroscopy is difficult and impractical when the cyst fluid is retained in amorphous stromal cells, or the like.

Therefore, the inventors of the present application conducted further consideration, and created a simulated living body sample (simulated cyst) that mimics the living tissue such as stromal cells, repeated spectroscopic measurements with respect to the simulated living body sample, and then analyzed the acquired optical spectra. This allowed a measuring method according to the present embodiment to be conceived of, which can measure the biomarker concentration with high accuracy by using absorbance at specific wavelengths even if the thickness, composition, moisture content, and optical path length of the living tissue are different.

In addition, it has previously been considered that it is difficult for visible light to reach into the cyst because of its low living body transmissibility, and the visible light is thus not very suitable for non-destructive testing; however, the inventors of the present application have found that if the visible light has a sufficient light amount to reach the cyst fluid retained in stromal cells, and the reflected light or transmitted light can be detected, it is possible to acquire an intrinsic light signal therefrom. Such efforts of the inventors of the present application have allowed the biomarker concentration in the cystic fluid retained in an endometriotic ovarian cyst to be measured, in situ, in a non-invasive manner and with good accuracy by using light of a specific wavelength in the wavelength region ranging from visible light to near-infrared light.

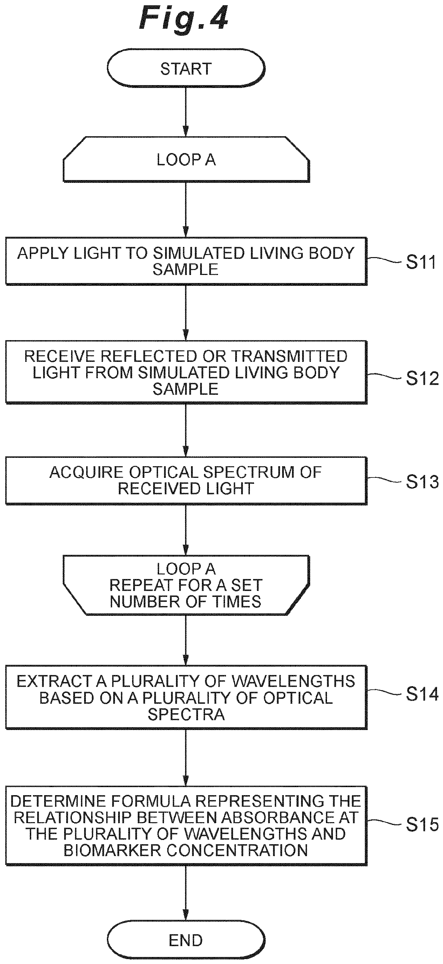

FIG. 4 is a flowchart showing the method of determining the formula used in the concentration calculation step S5 shown in FIG. 3.

First, a plurality of simulated living body samples are created, in each of which a transparent container containing a fluid of a known biomarker (hemoglobin in the present embodiment) concentration is covered by the living tissue, and the processing in steps S11 to S13 is repeated the number of times set for each simulated living body sample. The fluid may be any fluid with a known hemoglobin concentration, and may be, for example, hemoglobin aqueous solution obtained by dissolving hemoglobin powder in pure water, or a cystic fluid collected from a living body. In the latter case, the hemoglobin concentration should be measured beforehand using well-known concentration measuring means, such as a spectrophotometer.

The container for containing the fluid is not particularly limited if it is formed of transparent and uniform material. Preferably, transparent cells and cuvettes used in general spectrophotometers, or the like, may be used. In addition, commercially available tissues, such as pork, chicken or fats thereof, may be used as the living body tissue for wrapping the container.

Light in the wavelength region ranging from visible light to near-infrared light is applied to such simulated living body sample (step S11). The light applied may be visible light only, near-infrared light only, or visible light and near-infrared light. In accordance with the invention the components of the plurality of wavelengths include, at least, components of a wavelength in a visible light region. There is no problem even if the light applied contains light with wavelengths beyond the wavelength region ranging from visible light to near-infrared light (e.g., ultraviolet light). A light source that generates light to be applied is not particularly limited, and, for example, a halogen lamp may be used.

Next, the reflected light reflected by the simulated living body sample or transmitted light transmitted through the simulated living body sample is received (step S12). Further, an optical spectrum of the received light is acquired (step S13). The means for receiving the reflected or transmitted light to acquire the optical spectrum is not particularly limited, and general spectrometers, spectroscopic sensors, and the like, may be used.

By repeating these steps S 11-S13, a plurality of optical spectra corresponding to a plurality of simulated living body samples are acquired. Then, based on the plurality of acquired optical spectra, a plurality of wavelengths, in which characteristic light signals can be observed, are extracted from the wavelength region of the light applied in step S11 (step S14). It can be said that this characteristic light signal indicates the presence of biomarkers.

An example of a wavelength extraction method will now be described. First, second derivative is performed on each optical spectrum. The second derivative method is not particularly limited, and, for example, the Savitzky-Golay method may be used. In the second derivative of a spectrum, a significant difference in the original spectrum appears as a downward peak, so the wavelength at which the downward peak appears is extracted. In this case, all downward peaks may be extracted, or a predetermined number of wavelengths may be selected from the largest peak. Alternatively, all wavelengths with peaks equal to or greater than a predetermined value (absolute value) may be selected.

Then, a formula representing the relationship between absorbance at the plurality of wavelengths selected in step S14 and biomarker concentration is determined (step S15). Specifically, absorbance at the selected wavelengths is acquired based on each of the plurality of optical spectra, and regression analysis is performed using the acquired absorbance as an explanatory variable and the known biomarker concentration in the solution in the simulated living body samples as a response variable. The regression analysis method is not particularly limited if analysis is possible according to the number of samples (number of measurements) and the number of selected wavelengths. An example of an applicable regression analysis includes partial least squares (PLS) regression analysis, which is a type of multivariate analysis.

It should be noted that step S14 described above is not essential. If step S14 is omitted, similar regression analysis may be performed in step S15 by using absorbance at all wavelengths detectable in the wavelength region of the applied light.

The formula thus determined is less susceptible to factors, such as the thickness, composition, and moisture content of the living tissue, and differences in optical path lengths arising from these factors, and accurately reflects the biomarker concentration to be measured. Accordingly, by substituting the absorbance measurement values at specific wavelengths, which are acquired by applying light to the ovarian cyst transvaginally or transabdominally, into the above-described formula (see the concentration calculation step S5 in FIG. 3), the hemoglobin concentration in the cystic fluid retained in the ovarian cyst may be measured with quantitatively high accuracy without being greatly affected by the thickness or composition of stromal cells or visceral fat.

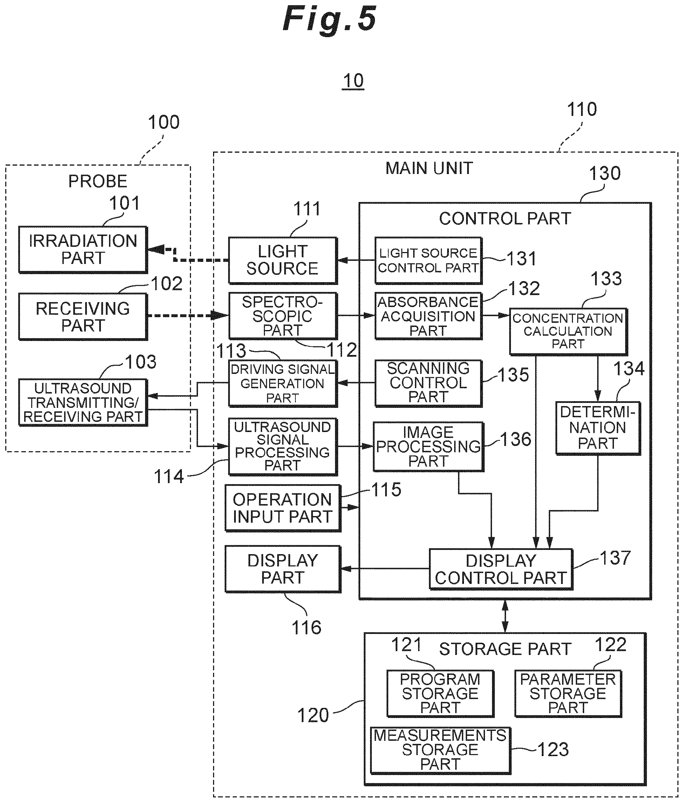

FIG. 5 is a block diagram illustrating a schematic configuration of a hemoglobin concentration measuring system according to the present embodiment of the present invention. The hemoglobin concentration measuring system shown in FIG. 5 also has a function of determining the degree of malignancy of an endometriotic ovarian cyst using hemoglobin in the cystic fluid as a biomarker. The hemoglobin concentration measuring system 10 shown in FIG. 5 is equipped with a transvaginal or transabdominal probe 100 and a main unit 110. The probe 100 and the main unit 110 are connected by a cable containing a light guide fiber and a wire for electrical signal transmission.

The probe 100 includes: an irradiation part 101 that applies light to living tissue of a subject (patient), the light being transmitted from the main unit 110 via a light guide fiber for light transmission; a light receiving part 102 that receives reflected light reflected from the living tissue or transmitted light transmitted through the living tissue and transmits it to the main unit 110 via a light guide fiber for receiving light; and an ultrasound transmitting/receiving part 103 that transmits ultrasound to the living tissue and receives ultrasound echoes reflected from the living tissue.

The irradiation part 101 may be a part having an optical system, such as a focus lens, provided at an end of the light guide fiber. In addition, the light receiving part 102 may be a part having an optical system, such as a collector lens that collect reflected or transmitted light, provided at an end of the light guide fiber.

The ultrasound transmitting/receiving part 103 is configured by using one or more piezoelectric oscillators with electrodes formed at both ends of a piezoelectric body, such as piezoelectric ceramics, such as PZT (lead zirconate titanate), and polymeric piezoelectric elements, such as PVDF (polyvinylidene fluoride). The ultrasound transmitting/receiving part 103 generates ultrasound based on driving electrical signals transmitted from the main unit 110, and receives ultrasound echoes reflected from the living tissue, converts them into electrical signals (ultrasound reception signals), and transmits them to the main unit 110.

The main unit 110 is equipped with: a light source 111 that generates light to be transmitted to the probe 100; a spectroscopic part 112 that receives and disperses the light transmitted from the probe 100; a driving signal generation part 113 that generates an ultrasound driving signal and transmit it to the probe 100; an ultrasound signal processing part 114 that processes the ultrasound reception signal transmitted from the probe 100; an operation input part 115; a display part 116; a storage part 120; and a control part 130.

The light source 111 generates light that contains components of a plurality of specific wavelengths of the wavelength region ranging from visible light to near-infrared light. Preferably, the light source 111 generates light with good directionality. As an example, the light source 111 generates light containing components of two or more wavelengths selected from among 580 nm, 590 nm, 640 nm, 680 nm, 762 nm, 876 nm, 900 nm, 932 nm, 958 nm, 968 nm, 978 nm, and 1095 nm. As the light source 111, a light source combining multiple LEDs with different peak wavelengths may be used, or a halogen lamp that generates light ranging from ultraviolet light to near-infrared light may be used. The light generated by the light source 111 is transmitted to the irradiation part 101 via the light guide fiber.

The spectroscopic part 112 acquires an optical spectrum of the light received by the light receiving part 102 and transmitted via the light guide fiber, and outputs a signal representing the intensity of each wavelength component contained in such light. A general spectrometer and a spectroscopic sensor may be used as the spectroscopic part 112. For example, a combination of multiple sensors that are sensitive only to specific wavelengths may be used, or a sensor that is capable of detecting a wide wavelength region may be used.

The driving signal generation part 113 may be configured by, for example, a pulser, and generates a driving signal to be applied to one or more piezoelectric oscillators configuring the ultrasound transmitting/receiving part 103.

The ultrasound signal processing part 114 may be configured by, for example, an amplifier and an A/D converter, and produces a digital ultrasound reception signal by applying signal processing, such as amplification and A/D conversion, to the ultrasound reception signal transmitted from the ultrasound transmitting/receiving part 103.

The operation input part 115 may be configured by using, for example, input devices, such as an operation button, operation lever, keyboard, mouse, and touch panel, and inputs a signal corresponding to external operations to the control part 130.

The display part 116 may be, for example, a liquid crystal display or an organic EL display, and displays, under the control of the control part 130, ultrasound images based on the ultrasound reception signals and/or information such as biomarker concentration measurements.

The storage part 120 may be, for example, a computer readable storage medium such as a hard disk and/or a solid-state memory, such as a ROM or RAM, and includes a program storage part 121, a parameter storage part 122, and a measurement storage part 123.

The program storage part 121 stores, in addition to operating system programs and driver programs for operating the control part 130, application programs for performing various functions. In particular, the program storage part 121 stores a measurement program for causing the control part 130 to perform an operation of measuring biomarker concentration based on the optical spectra acquired by the spectroscopic part 112, a determination program for causing the control part 130 to perform an operation of determining the degree of malignancy of endometriotic ovarian cysts based on the biomarker concentration, and other programs

The parameter storage part 122 stores various parameters, and the like, to be used during execution of the programs stored in the program storage part 121. For example, the parameter storage part 122 may store the parameters of the formula used during the execution of the biomarker concentration measurement program. This formula is a formula representing the relationship between absorbance at a plurality of specific wavelengths and the biomarker concentration in the living body, and may be represented by the above-described formula (2). The parameter storage part 122 may store the coefficients $a_n$ and the constant b configuring the formula (2). The parameter storage part 122 also stores the cutoff values of hemoglobin concentration used in the program for determining the degree of malignancy of endometriotic ovarian cysts.

The measurement storage part 123 stores measurement results, such as measurement values of biomarker (hemoglobin) concentration and the degree of malignancy of endometriotic ovarian cysts.

The control part 130 may be configured by using a central processing unit (CPU), and controls the respective parts in the hemoglobin concentration measuring system 10 in an integrated manner by reading various programs stored in the program storage part 121 and performs various types of computational processing in order to measure biomarker concentration and to determine the degree of malignancy of endometriotic ovarian cysts based on the measurements. Specifically, functional parts implemented by the control part 130 include a light source control part 131, an absorbance acquisition part 132, a concentration calculation part 133, a determination part 134, a scanning control part 135, an image processing part 136, and a display control part 137.

The light source control part 131 controls switching on/off of the light source 111, light application time, light application intensity, and the like, according to the signals input from the operation input part 115. In the present embodiment, only optical spectra of the reflected light or transmitted light from the living tissue need to be acquired, so the light application time in a single measurement is sufficient at the level of milliseconds at most.

The absorbance acquisition part 132 acquires, based on the signals representing the intensities of the wavelength components output from the spectroscopic part 112, the absorbance measurement value at such wavelengths. The absorbance may be calculated using the above-described formula (1) based on the Beer-Lambert law.

The concentration calculation part 133 calculates the biomarker concentration by substituting the absorbance measurement values acquired by the absorbance acquisition part 132 into the formula configured by using the parameters (e.g., the coefficient $a_n$ and constant b in the above-described formula (2)) stored in the parameter storage part 122.

The determination part 134 determines the degree of malignancy of endometriotic ovarian cysts by comparing the biomarker concentration calculated by the concentration calculation part 133 to the cutoff values stored in the parameter storage part 122. For example, the determination part may determine the occurrence of carcinogenesis with a probability of 85.7% when the hemoglobin concentration in the cystic fluid is between 0.0 and 2.0 g/dL.

The scanning control part 135 scans the subject with ultrasound transmitted from the ultrasound transmitting/receiving part 103 by controlling the occurrence timing of the driving signals (delay pattern of signals) in the driving signal generation part 113. The ultrasound scanning pattern is not particularly limited, and it may be set as appropriate according to the probe shape, the scanning target area, and the like, and includes radial scanning, sector scanning, and other scanning.

The image processing part 136 produces an ultrasound image based on the digital ultrasound reception signals produced by the ultrasound signal processing part 114.

The display control part 137 controls the display part 116 so that predetermined information, such as information input through the operation input part 115 and information processed in the control part 130, is displayed in a predetermined format. For example, the display control part 137 may display the ultrasound image generated by the image processing part 136 on the display part 116, and superimpose the direction of light emitted from the irradiation part 101 of the probe 100 on the ultrasound image by animation. This allows a user operating the hemoglobin concentration measuring system 10 to locate the measurement target of biomarker concentration (e.g., the ovarian cyst) and to reliably apply light to the measurement target. In addition, the display control part 137 may perform control so that the information representing the biomarker concentration calculated by the concentration calculation part 133 is superimposed on a target region for measuring biomarker concentration in the ultrasound image. As a specific example, the region of the ovarian cyst in the ultrasound image may be superimposed with color or brightness shading according to the biomarker concentration values. In addition, the display control part 137 may display the determination result by the determination part 134 and/or an alarm corresponding to this determination result on the display part 116.

The main unit 110 may be configured by a single device, or by a plurality of devices connected via a telecommunications cable or network.

In addition, in the present embodiment, the irradiation part 101 and the light receiving part 102 are provided on the probe 100 side, and the light source 111 and the spectroscopic part 112 are provided on the main unit 110 side, but the light source 111 and/or the spectroscopic part 112 may be provided on the probe 100 side.

In addition, in the present embodiment, the irradiation part 101 and the light receiving part 102 are provided on the same probe, but they may be provided on separate probes. For example, by providing a transvaginal probe with the light receiving part 102 and applying light from the abdominal surface, the transmitted light that transmitted through the ovarian cyst may be received in the vagina. Alternatively, by providing the transvaginal probe with the irradiation part 101 and applying light from within the vagina, the transmitted light that transmitted through the ovarian cyst may be received at the abdominal surface.

In the present embodiment, the hemoglobin concentration measuring system is combined with ultrasound image generation means, but it may be combined with medical image generation means other than the ultrasound image, such as an MRI. In this case, the hemoglobin concentration measurements obtained by the system according to the present embodiment may be superimposed on a medical image generated by the medical image generation means.

In addition, in the present embodiment, the degree of malignancy of endometriotic ovarian cysts is determined by comparing the hemoglobin concentration obtained as the measurements with a preset cutoff values, but the degree of malignancy may further be determined in combination with data such as the age of the subject (patient) or the size of the cyst. The size of the cyst may be measured based on ultrasound images and/or MRI images. For example, from an epidemiological point of view, it is known that, if the patient is 44 years old or over, or if the maximum cyst diameter is 84 mm or larger, the possibility of the endometriotic ovarian cyst being cancerous is high, so the cutoff values may be adjusted according to the patient's age and/or the size of the cyst.

As described above, according to the present embodiment, the biomarker concentration is calculated by substituting the absorbance measurement values at a plurality of specific wavelengths acquired by applying light to the living tissue into a pre-acquired formula that represents the relationship between absorbance at the plurality of specific wavelengths and biomarker concentration. Therefore, the biomarker concentration in the fluid retained in a living tissue can be measured quantitatively, in situ, in a non-invasive manner with good accuracy. In addition, since the above-described formula is pre-acquired, the measurement can be performed rapidly and in real time. In addition, since the biomarker concentration can be measured in an unrestrained, painless or reduced pain, and non-invasive manner for the subject, examinations with less physical burden can be performed.

In addition, according to the present embodiment, when measuring the hemoglobin concentration in the cystic fluid, two or more wavelengths selected from among the 12 wavelengths of 580 nm, 590 nm, 640 nm, 680 nm, 762 nm, 876 nm, 900 nm, 932 nm, 958 nm, 968 nm, 978 nm, and 1095 nm are used. Therefore, light signals can be acquired that are less susceptible to factors such as: the thickness, composition, and moisture content of the living tissue, such as stromal cells and visceral fat, that is present around the cystic fluid; and differences in optical path lengths, while accurately reflecting the hemoglobin concentration. Accordingly, the hemoglobin concentration can be accurately measured regardless of differences in thickness, composition, and the like, of stromal cells. In addition, since the above-described 12 wavelengths are in the wavelength region ranging from visible light to near-infrared light, highly safe examinations can be conducted.

In addition, according to the present embodiment, since the hemoglobin concentration can be measured using two or more wavelengths selected from the above-described 12 wavelengths, a light source can be configured by combining multiple LEDs with different peak wavelengths. In other words, the degree of freedom in designing of optics can be increased.

In the present invention, a multiple regression formula is constructed based on measurements using simulated cysts. Therefore, when measuring the hemoglobin concentration in the cystic fluid in vivo, the effects of the tissues, such as stromal cells and visceral fat, can be reduced by using this multiple regression formula. However, there is also an environment different from that in the laboratory within the living body, such as the body temperature of the subject (patient). It is difficult to reproduce the environments in the living bodies of the individual subjects in the laboratory and construct a multiple regression formula based on measurements using simulated cysts under such environments. In this respect, since visible light is relatively less susceptible to the temperature effects, the effects of the differences in environments, such as temperature, on the measurement values can be reduced by using visible light and near-infrared light, or only visible light in the measurement using the simulated cysts and in vivo measurement of the subjects. This allows the hemoglobin concentration in the cystic fluid to be measured stably in vivo based on the multiple regression formula obtained by the measurement using the simulated cysts.

In addition, according to the present embodiment, since the hemoglobin concentration can be measured using absorbance of at least two wavelengths, the computational load is small. Therefore, the measurements can be obtained rapidly even when a device with specifications comparable to a commercial-off-the-shelf personal computer is used. Accordingly, downsizing of the system and reduction in manufacturing and operating costs can be achieved.

In addition, according to the present embodiment, since the hemoglobin concentration in the cystic fluid retained in the ovarian cyst can be accurately measured, in situ, in a non-invasive manner, the degree of malignancy can be reliably determined in a simple and non-invasive manner for a frequent number of times by referring to the classification or cutoff values of the degrees of malignancy of endometriotic ovarian cysts pre-associated with hemoglobin concentration. Accordingly, for patients diagnosed with endometriotic ovarian cysts, there is a possibility of reducing unnecessary surgeries, such as removing the ovary in consideration of the possibility of the ovarian cyst developing into cancer in the future, despite it not being cancerous at the current time, and alleviating the physical and financial burden on the patients.

In addition, according to the present embodiment, there is no need to take the exposure to X-ray and/or the side effects caused by contrast agents into consideration when determining the degree of malignancy of endometriotic ovarian cysts. In addition, in the present embodiment, since the measurement time is at the level of milliseconds, the effects on the living body are small even if a light source containing ultraviolet light is used. Accordingly, there is less physical burden on the patients and examinations can be conducted frequently.

In addition, according to the present embodiment, the degree of malignancy of endometriotic ovarian cysts can be determined in a simple and rapid manner without using any large instruments, such as an MRI. Accordingly, the present embodiment can find application in examinations in medical inspections in small-scaled clinics and in regular medical inspections. In particular, by using the hemoglobin concentration measuring system as illustrated in FIG. 5, it is possible to automatically perform the procedure from measurement of the hemoglobin concentration to determination of the degree of malignancy. Accordingly, there is a possibility of increasing the variations of treatment regimens, such as delivering medication according to follow-ups and/or determination results, and applying appropriate surgical treatment at the stage when it becomes necessary to do so.

It should be noted that the method of determining the degree of malignancy of endometriotic ovarian cysts described in the present embodiment does not constitute a definite diagnosis of whether an endometriotic ovarian cyst has become cancerous. The definite diagnosis of whether an endometriotic ovarian cyst has become cancerous will be provided histopathologically.

(Examples)

An experiment was conducted in which a formula was created, which is to be used in calculating the hemoglobin concentration based on the optical spectrum of the transmitted light from a simulated living body sample that mimics an ovarian cyst (hereafter referred to as a "simulated cyst"), and the accuracy of the hemoglobin concentration calculated using this formula was verified.

Example 1-1

(1) Creation of specimen (simulated cyst)

Hemoglobin aqueous solutions were prepared by dissolving hemoglobin powder (manufactured by Sysmex Corporation, hemolytic hemoglobin) in pure water. Six different hemoglobin concentrations were prepared: 0.0 g/dL (pure water only), 0.5 g/dL, 1.0 g/dL, 2.0 g/dL, 3.0 g/dL, and 4.0 g/dL. These concentrations were in the concentration range around the hemoglobin concentration of 2.0 g/dL, which was the cutoff value of the degree of malignancy of endometriotic ovarian cysts. The hemoglobin concentrations were actually measured using a hematology analyzer (manufactured by Sysmex Corporation, type name: XN-330).

4 mL of each hemoglobin aqueous solution with a corresponding concentration was injected into a disposable polystyrene cuvette (manufactured by Bio-Rad, internal dimensions: 10 mm x 10 mm x 45 mm) and two glass cuvettes of different sizes (manufactured by Tokyo Glass Kikai Co., Ltd., internal dimensions: 10 mm x 10 mm x 45 mm, 20 mm x 10 mm x 45 mm), and then sealed with parafilms. Then, a simulated cyst was created by placing the container encapsulating the hemoglobin aqueous solution on its side and wrapping it with pork. Two different thicknesses of pork were provided, 5 mm and 10 mm, at the section where an optical fiber (described below) makes contact with the pork. As specimens, a total of 36 simulated cysts were prepared with two different pork thicknesses and three different containers for each of the six different hemoglobin concentrations above.

(2) Light application to simulated cysts and acquisition of absorbance

Two optical fibers for near-infrared light (manufactured by Hamamatsu Photonics K.K., type names: A7969-08AS and A9763-01) were arranged in parallel so that the tip surfaces are in the same plane and the center-to-center distance is 18 mm, and fiber holders (manufactured by Thorlabs Inc., ADASMAB2) were used to fix them. Then, the positions of the fiber holders and a specimen stage were adjusted so that the tip surfaces of the two optical fibers were in contact with the simulated cyst to be placed on the specimen stage, and the fiber holders and the specimen stage were fixed to an optical test bench so that the optical axis did not shift during the measurement, and thereby the measuring optical system was installed.

In addition, a halogen lamp (manufactured by Hamamatsu Photonics K.K., High Power UV/Vis, type name: L10290) was connected, as a light source, to the rear end of one optical fiber (A9763-01), and a spectrometer (manufactured by Hamamatsu Photonics K.K., type name: C9405CB) was connected to the rear end of the other optical fiber (A7969-08AS). Further, a black polypropylene board was processed to create a dark box, which was then placed over the measuring optical system, so that no extra light, such as sunlight or lighting, could enter from the outside.

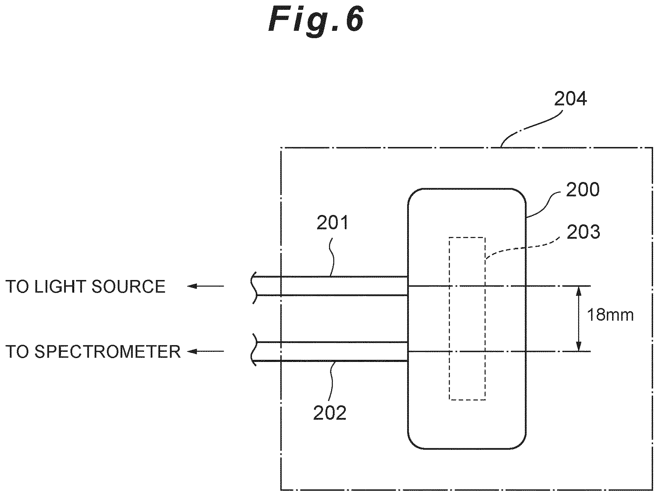

FIG. 6 is a plan view schematically showing the above-described measuring optical system. When the tip surfaces of the two optical fibers 201 and 202 come into contact with the simulated cyst 200, the light emitted from the tip surface of one optical fiber 201 enters the interior of the simulated cyst 200. This light is reflected in the simulated cyst 200, and part of the reflected light enters the tip surface of the other optical fiber 202. The light that entered this optical fiber 202 includes information about the fluid contained in the container 203 in the simulated cyst 200. In FIG. 6, the dark box 204 is shown by a dashed-dotted line. In addition, the simulated cyst was covered with food plastic wrap to prevent staining during the actual measurement.

First, after the light source and spectrometer were turned on and energized for more than 30 minutes, an optical spectrum was acquired with nothing placed on the specimen stage and with the dark box placed thereon, and the light intensity $I_0$ at each wavelength was measured. Next, a simulated cyst was placed on the specimen stage and an optical spectrum was acquired, and the light intensity $I_1$ at each wavelength was measured. The number of measurement points (wavelengths) was set to 1025 points (wavelengths) that can be detected in the range of 434 nm to 1144 nm, based on the specifications of the spectrometer. Then, based on the light intensities $I_0$ and $I_1$, absorbance at each wavelength was calculated according to the Beer-Lambert law. Such measurement was performed four times (i.e., a total of 144 times) for the simulated cysts with six different concentrations, two different thicknesses, and three different containers.

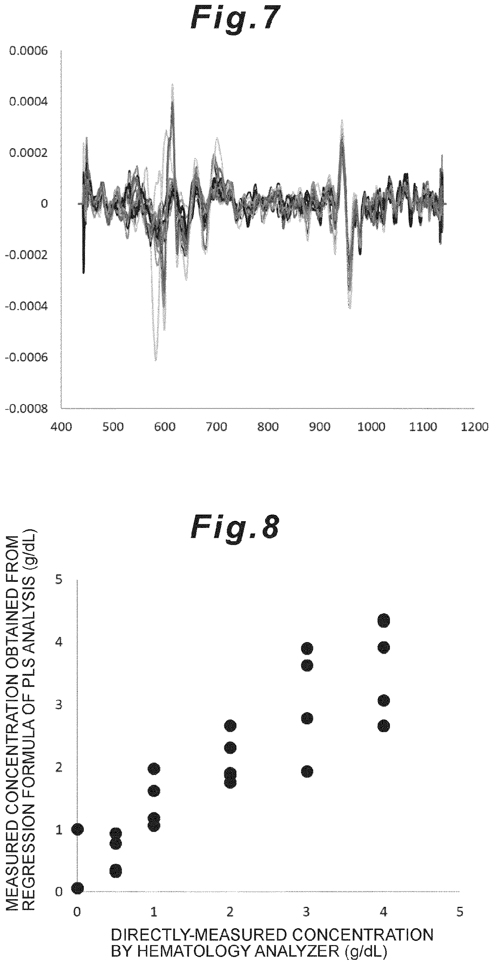

FIG. 7 is a graph showing the second derivative of the optical spectra obtained in each measurement. The Savitzky-Golay method was employed as the second derivative method. Here, in the second derivative of the optical spectrum, the significant difference in the original spectrum appears as a downward peak. As shown in FIG. 7, in the present example, peaks that are clearly not noise can be observed at the positions of 12 wavelengths of 580 nm, 590 nm, 640 nm, 680 nm, 762 nm, 876 nm, 900 nm, 932 nm, 958 nm, 968 nm, 978 nm, and 1095 nm. These peaks are considered to characterize the hemoglobin concentration in the simulated cyst.

(3) Creation and verification of regression formula

Out of the 144 measurements (absorbance at 1025 points (wavelengths) per measurement) obtained by the above measurement, 30 measurements were randomly extracted and excluded, and the remaining 114 measurements were used for regression analysis to acquire a regression formula. As for the regression analysis, PLS analysis, which is a type of multivariate analysis, was performed using the absorbance at all wavelengths at 1025 points as an explanatory variable, and the hemoglobin concentration obtained by actual measurement with the hematology analyzer as a response variable.

The hemoglobin concentration was calculated by substituting each of the 30 excluded measurements (same as above) into the regression formula obtained. Then, the correlation between the hemoglobin concentration calculated by the substitution into the regression formula and the hemoglobin concentration obtained by directly measuring the hemoglobin aqueous solution with the hematology analyzer was determined.

FIG. 8 is a graph showing the correlation between the hemoglobin concentration measured directly by the hematology analyzer (hereinafter referred to as the "directly-measured concentration") and the measured hemoglobin concentration obtained from the regression formula (hereinafter referred to as the "calculated concentration"). The correlation coefficient between the directly-measured concentration by the hematology analyzer and the concentration calculated by the regression formula was R = 0.91, and it could be recognized that there was a high correlation between the two hemoglobin concentrations. Based on the above, it was found that the hemoglobin concentration in the aqueous solution inside the simulated cyst wrapped in meat could be accurately measured without removing it from meat. It was also found that the hemoglobin concentration could be calculated by creating a unified regression formula for various simulated cysts with different meat thicknesses and cuvette sizes.

Patients with endometriotic ovarian cysts individually vary in cell thickness, visceral fat and muscle tissue density, tumor size, and the like. In the measuring method in the present example, the hemoglobin concentration in the cystic fluid in the ovarian cyst can be calculated by applying the unified regression formula to the patients with various different conditions as described above, and it can be said that it is an extremely versatile measuring method.

Example 1-2

A regression formula was acquired by performing regression analysis as with Example 1-1 above using the measurements obtained from the experiment in Example 1-1, and the hemoglobin concentration was calculated by this regression formula. However, the absorbance at 12 wavelengths of 580 nm, 590 nm, 640 nm, 680 nm, 762 nm, 876 nm, 900 nm, 932 nm, 958 nm, 968 nm, 978 nm, and 1095 nm, in which significant downward peaks can be observed in the second derivative of the optical spectra (see FIG. 7), was used as the explanatory variable instead of the 1025 points (wavelengths).

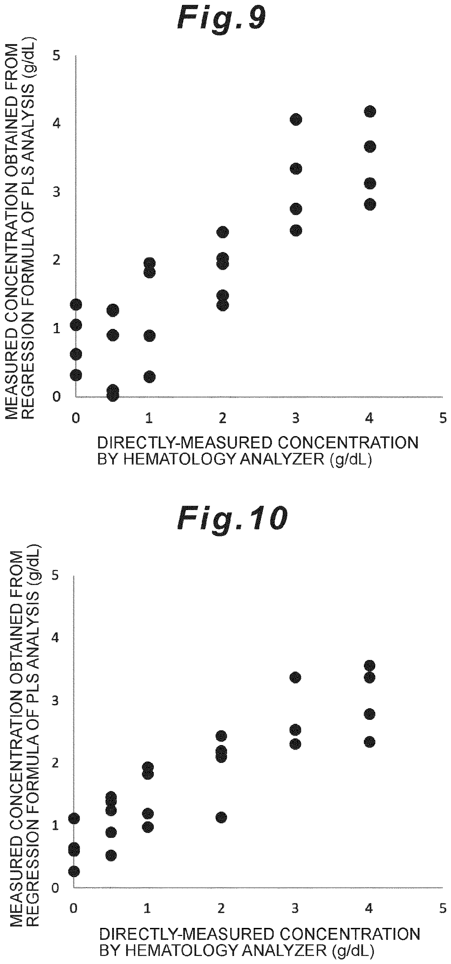

FIG. 9 is a graph showing the correlation between the hemoglobin concentration measured directly by the hematology analyzer (directly-measured concentration) and the measured hemoglobin concentration calculated by the regression formula using absorbance at the 12 wavelengths (calculated concentration). The correlation coefficient between the directly-measured concentration by the hematology analyzer and the concentration calculated by the regression formula was R = 0.88. Thus, even when the explanatory variable were limited to the absorbance at the above-described 12 wavelengths, it could still be recognized that there is a good correlation between the directly-measured hemoglobin concentration by the hematology analyzer and the calculated hemoglobin concentration by the regression formula.

Example 1-3

A regression formula was acquired by performing a regression analysis as with Example 1-1 above using the measurements obtained from the experiment in Example 1-1, and the hemoglobin concentration was calculated by this regression formula. However, only absorbance at two wavelengths of 900 nm and 968 nm out of the 12 wavelengths, in which significant downward peaks can be observed in the second derivative of the optical spectra (see FIG. 7), was used as the explanatory variable.

FIG. 10 is a graph showing the correlation between the hemoglobin concentration measured directly by the hematology analyzer (directly-measured concentration) and the measured hemoglobin concentration calculated by the regression formula using absorbance at the 2 wavelengths (calculated concentration). Even when the explanatory variable were limited to the absorbance at the above-described 2 wavelengths, the correlation coefficient between the directly-measured concentration by the hematology analyzer and the calculated concentration by the regression formula was R = 0.89, and it could therefore still be recognized that there is a good correlation between the two hemoglobin concentrations.

Example 1-4

A regression formula was acquired by performing regression analysis as with Example 1-1 above using the measurements obtained from the experiment in Example 1-1, and the hemoglobin concentration was calculated by this regression formula. However, the absorbance at any two or more wavelengths from the 12 wavelengths of 580 nm, 590 nm, 640 nm, 680 nm, 762 nm, 876 nm, 900 nm, 932 nm, 958 nm, 968 nm, 978 nm, and 1095 nm, in which significant downward peaks can be observed in the second derivative of the optical spectra (see FIG. 7), was used as the explanatory variable.

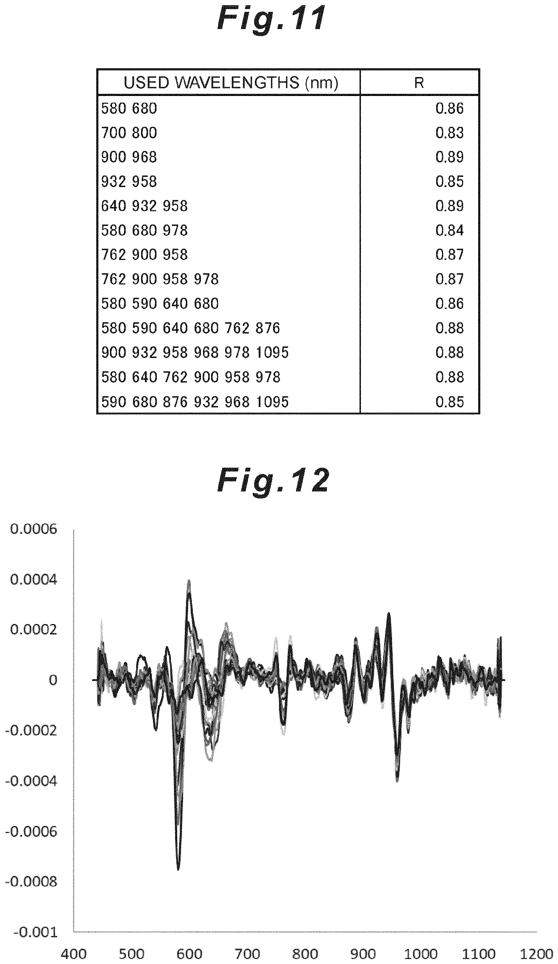

FIG. 11 is a table showing the wavelengths of the absorbance used in the creation of the regression formulas and the correlation coefficients between the calculated hemoglobin concentration by such regression formulas and the directly-measured hemoglobin concentration by the hematology analyzer. As shown in FIG. 11, the correlation coefficient in any of the wavelength combinations is equal to or greater than R = 0.83, and a good correlation can therefore be observed between the two hemoglobin concentrations. As a result, it was found that a regression formula that can accurately calculate the hemoglobin concentration can be created by using absorbance at any two or more wavelengths out of the above 12 wavelengths.

In addition, since visible light does not transmit through living bodies, near-infrared light has conventionally been used exclusively for spectroscopic measurements of the living bodies. But, as shown in FIG. 11, it was found that even when using only visible light or visible light and near-infrared light, accuracy equivalent to or greater than that of using only near-infrared light could be obtained.

Example 2-1

(1) Creation of specimen (simulated cyst)

Analytes (cyst fluids) provided from nine patients who underwent oophorectomy were prepared. When the hemoglobin concentration of these analytes was actually measured using a hematology analyzer (manufactured by Sysmex Corporation, type name: XN-330), it was in the range between 0.1 g/dL and 3.8 g/dL. A total of 10 analytes were prepared, using a solution obtained by dissolving human-derived albumin (manufactured by Nakalai Tesque, Inc.) in phosphate buffered saline (albumin concentration of 5.0 g/dL) as a blank with a hemoglobin concentration of 0.0 g/dL.

As with Example 1-1, simulated cysts were created by injecting 4 mL of these analytes into disposable polystyrene cuvettes (manufactured by Bio-Rad, internal dimensions: 10 mm x 10 mm x 45 mm), sealing them with parafilms, and wrapping them with pork. Two different thicknesses of pork were provided, 5 mm and 10 mm, at the section where an optical fiber makes contact with the pork. As specimens, a total of 20 simulated cysts were prepared with two different pork thicknesses for each of the ten analytes above.

(2) Light application to simulated cysts and acquisition of absorbance

A measuring optical system was installed as with Example 1-1 above, and an experiment of calculating absorbance by acquiring an optical spectrum of the light transmitted through the simulated cyst was conducted twice for each specimen, i.e., a total of 40 times.

FIG. 12 is a graph showing the second derivative of the optical spectra obtained in each measurement. As shown in FIG. 12, as with Example 1-1, characteristic peaks can also be observed in the present Example at the positions of 12 wavelengths of 580 nm, 590 nm, 640 nm, 680 nm, 762 nm, 876 nm, 900 nm, 932 nm, 958 nm, 968 nm, 978 nm, and 1095 nm. Taking into account Example 1-1 and Example 2-1, these peaks are considered to be peaks specific to the chemical species, i.e., hemoglobin, independent of the origin of the measurement target.

(3) Creation and verification of regression formula

Out of the 40 measurements (absorbance at 1025 points (wavelengths) per measurement) obtained by the above measurement, ten measurements were randomly extracted and excluded, and the remaining 30 measurements were used for regression analysis to acquire a regression formula. As with Example 1-1, as for the regression analysis, PLS analysis was performed using the absorbance at all wavelengths at 1025 points as an explanatory variable, and the hemoglobin concentration obtained by actual measurement with the hematology analyzer as a response variable.

The hemoglobin concentration was calculated by substituting each of the ten excluded measurements (same as above) into the regression formula obtained. Then, the correlation between the hemoglobin concentration calculated by the substitution into the regression formula and the hemoglobin concentration obtained by directly measuring the analyte with the hematology analyzer was determined.

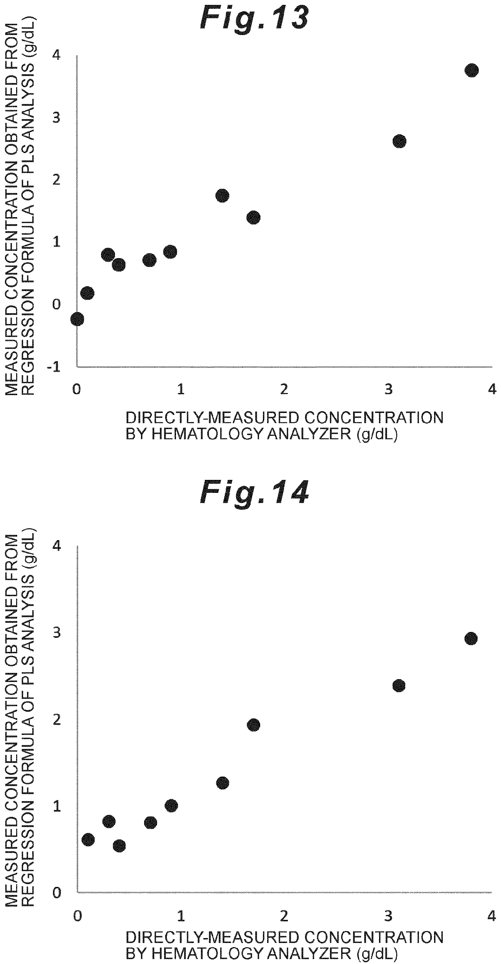

FIG. 13 is a graph showing the correlation between the hemoglobin concentration measured directly by the hematology analyzer (directly-measured concentration) and the measured hemoglobin concentration calculated by the regression formula (calculated concentration). The correlation coefficient between the directly-measured concentration by the hematology analyzer and the concentration calculated by the regression formula was R = 0.97, and it could be recognized that there was a high correlation between the two hemoglobin concentrations.

Thus, according to Example 2-1, it was found that the hemoglobin concentration can be accurately measured without necessitating removal from the simulated cyst, i.e., in a non-invasive manner with respect to not only the aqueous hemoglobin solution, but also the clinical analytes provided from the patients.

Example 2-2

A calibration curve was created from absorbance at two wavelengths of 900 nm and 968 nm out of the 30 measurements, excluding the 10 measurements from the measurements obtained by the experiment in Example 2-1 above. Then, the hemoglobin concentration was calculated by applying the measurements of the 10 excluded measurements to the calibration curve. FIG. 14 is a graph showing the correlation between the hemoglobin concentration measured directly by the hematology analyzer (directly-measured concentration) and the measured hemoglobin concentration calculated by the calibration curve determined from the PLS analysis (calculated concentration). The correlation coefficient between the directly-measured concentration by the hematology analyzer and the concentration calculated by the calibration curve was R = 0.92, and it could be recognized that there was a high correlation between the two hemoglobin concentrations.

Example 3

(1) Creation of specimen (simulated cyst)

As with Example 1-1, simulated cysts were created by encapsulating hemoglobin aqueous solutions, which were prepared in six different concentrations: 0.0 g/dL (pure water only), 0.5 g/dL, 1.0 g/dL, 2.0 g/dL, 3.0 g/dL, and 4.0 g/dL, in polystyrene cuvettes (manufactured by Bio-Rad, internal dimensions of 10 mm x 10 mm x 45 mm) and wrapping them with pork. A total of 12 simulated cysts were prepared by providing two different meat thicknesses, 5 mm and 10 mm.

(2) Light application to simulated cysts and acquisition of absorbance

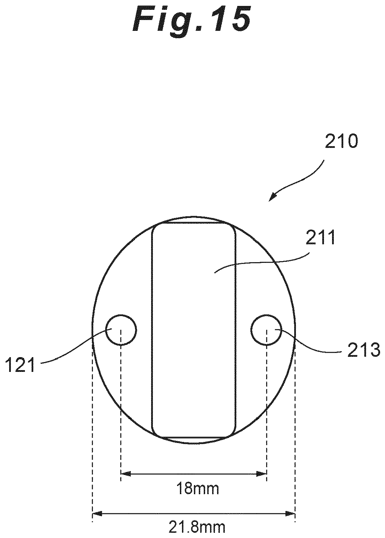

An experimental probe was created with an optical fiber embedded in the tip portion of the probe by using a body cavity probe (transvaginal probe) used for ultrasound diagnosis. FIG. 15 is a plan view schematically showing a tip portion (a head portion) of the probe used in Example 3. The head portion of the probe 210 is 21.8 mm wide, and an ultrasound transmitting/receiving part 211 is provided at the center of the head portion. An optical fiber 212 connected to a light source and an optical fiber 213 connected to a spectrometer were attached to such body cavity probe such that the tip surface of each optical fiber 212, 213 is flush with the surface of the head portion. The center-to-center distance between the optical fibers 212, 213 was 18 mm.

With respect to such experimental probe, ultrasound jelly (manufactured by JEX Co., Ltd.) was applied to the tip and a probe cover (manufactured by Fuji Latex Co., Ltd.) was placed thereover, as with the usual procedure for ultrasound diagnosis. The intention of following the usual procedure was to verify whether the light detection was still possible even if the light is attenuated by the ultrasonic jelly and the probe cover.

The experimental probe was fixed to an optical bench, and as with Example 1-1, the light intensity $I_0$ measured

without a simulated cyst on the specimen stage and the light intensity $I_1$ measured with a simulated cyst on the specimen stage were acquired. Then, based on the light intensities $I_0$ and $I_1$, absorbance at each wavelength was calculated according to the Beer-Lambert law. Such measurement was performed three times (i.e., a total of 36 times) for the simulated cysts with six different concentrations and two different meat thicknesses.

(3) Creation and verification of regression formula

Out of the 36 measurements (absorbance at 1025 points (wavelengths) per measurement) obtained by the above measurement, 9 measurements were randomly extracted and excluded, and the remaining 27 measurements were used for regression analysis to acquire a regression formula. As with Example 1-1, as for the regression analysis, PLS analysis was performed using the absorbance at all wavelengths at 1025 points as an explanatory variable, and the hemoglobin concentration obtained by actual measurement with the hematology analyzer as a response variable.

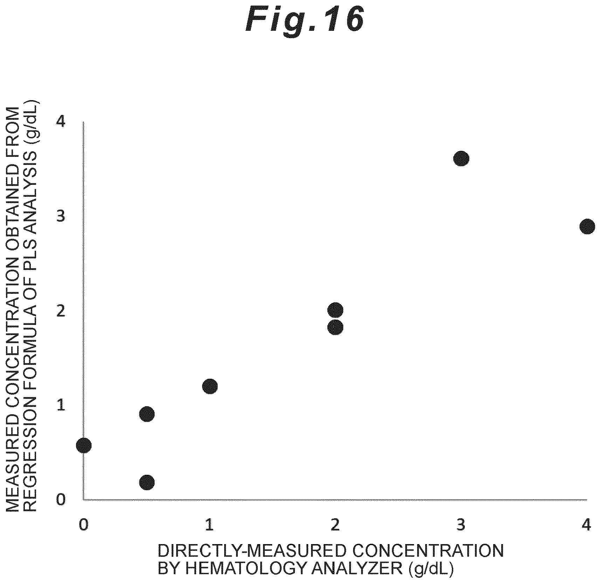

The hemoglobin concentration was calculated by substituting each of the 9 excluded measurements (same as above) into the regression formula obtained. Then, the correlation between the hemoglobin concentration calculated by the substitution into the regression formula and the hemoglobin concentration obtained by directly measuring the hemoglobin aqueous solution with the hematology analyzer was determined. FIG. 16 is a graph showing the correlation between the hemoglobin concentration measured directly by the hematology analyzer (directly-measured concentration) and the measured hemoglobin concentration calculated by the regression formula (calculated concentration). The correlation coefficient between the directly-measured concentration by the hematology analyzer and the concentration calculated by the regression formula was R = 0.89, and it could be recognized that there was a good correlation between the two hemoglobin concentrations.

Thus, it was found that the hemoglobin concentration in the fluid in the simulated cyst can still be accurately measured in a non-invasive manner by mounting an optical system, such as an optical fiber, on a body cavity probe, even when the measuring optical system is not configured with a special optical fiber holder for optical experiments. In addition, it was confirmed that light with a light amount sufficient for measuring hemoglobin concentration can still be detected even when such light has passed through the ultrasound jelly and the probe cover used in ultrasound diagnosis.

(Second Embodiment)

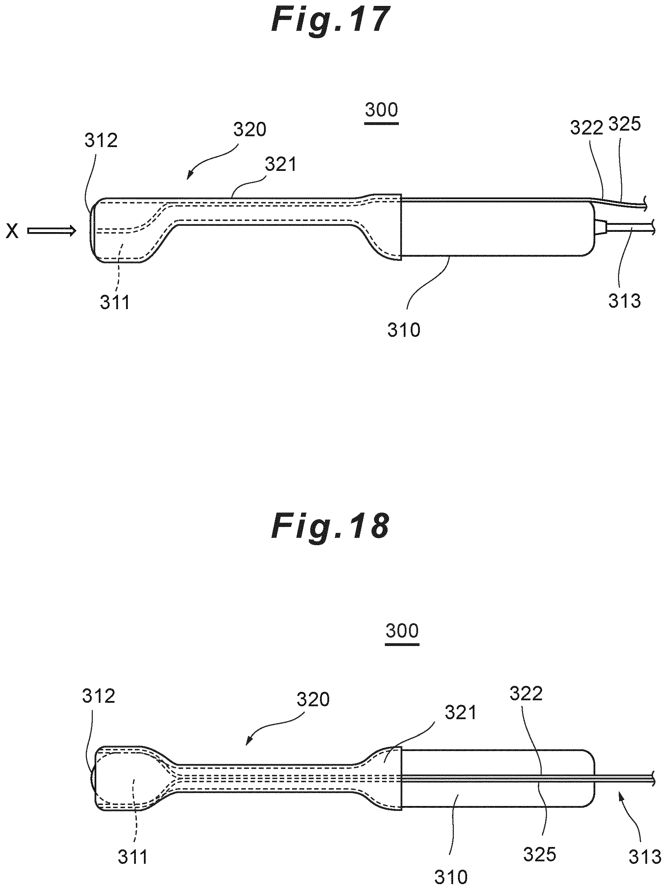

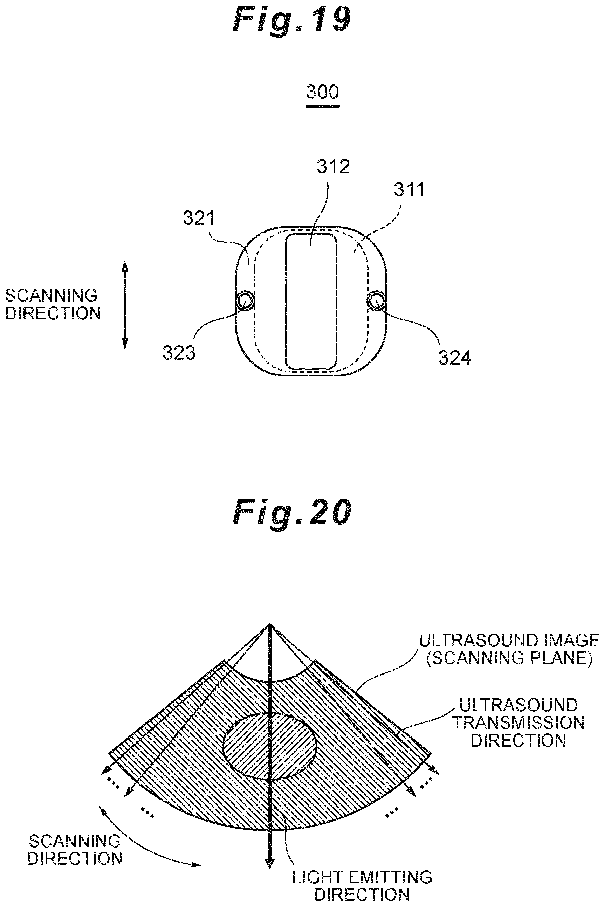

FIG. 17 is a side view schematically showing a transvaginal probe according to a second embodiment of the present invention. FIG. 18 is a top view of the transvaginal probe shown in FIG. 17. FIG. 19 is an enlarged view seen from arrow X in FIG. 17. FIG. 20 is a schematic diagram for describing the relationship between an ultrasound image and the direction of light application.

The transvaginal probe 300 shown in FIGS. 17 to 19 is a probe primarily used to measure hemoglobin concentration in the cystic fluid retained in an endometriotic ovarian cyst. The probe includes an ultrasound transmitting/receiving function to transmit ultrasound to living tissue of a subject (patient) and receive ultrasound echo reflected from the living tissue, a function to apply light to the living tissue, and a function to receive reflected light reflected from the living tissue or transmitted light transmitted through the living tissue. Such transvaginal probe 300 may be used by being connected to the main unit 110 of the hemoglobin concentration measuring system 10 shown in FIG. 5 and may configure part of the hemoglobin concentration measuring system.

Specifically, the transvaginal probe 300 is equipped with a transvaginal ultrasound probe 310 and an attachment 320 for light transmission/reception that can be attached to and detached from the ultrasound probe 310.

The tip portion of an insertion part 311, which is to be inserted in the vagina, of the ultrasound probe 310 is provided with an ultrasound transmitting/receiving part 312, which transmits ultrasound to the living tissue and receives ultrasound echo reflected from the living tissue. The ultrasound transmitting/receiving part 312 is configured using a plurality of piezoelectric oscillators arranged in a predetermined sequence. The ultrasound transmitting/receiving part 312 preferably scans the living tissue in a convex manner with the ultrasound transmitted from the tip portion of the insertion part 311. The ultrasound transmitting/receiving part 312 generates the ultrasound based on driving electrical signals transmitted from the main unit 110 connected to the ultrasound probe 310 via a cable 313, and receives the ultrasound echoes reflected from the living tissue, converts them into electrical signals (ultrasound reception signals), and transmits them to the main unit 110 via the cable 313.

The attachment 320 is equipped with: a holder 321 to be placed over the insertion part 311; a light guide fiber 322 for transmitting light, which is connected to the light source 111 of the main body 110; an irradiation part 323 for applying light to the living tissue of a subject, the light being transmitted through the light guide fiber 322; a light receiving part 324 for receiving reflected light reflected from the living tissue or transmitted light transmitted through the living tissue; and a light guide fiber 325 for receiving light, which is connected to the spectroscopic part 112 of the main unit 110 and transmits the light received by the light receiving part 324 to the spectroscopic part 112. The irradiation part 323 and the light receiving part 324, and at least part of the light guide fibers 322 and 325, are attached to the holder 321.

The holder 321 may be made of biocompatible resin materials, such as polyurethane, polyether ether ketone, polymethyl methacrylate, and polyisoprene. The raw material of the holder 321 is not limited if it exhibits biocompatibility that ensures the safety of the subject, regardless of the hardness and color of the material. The holder 321 fixes the irradiation part 323 and the light receiving part 324, and part of the light guide fibers 322, 325, to the insertion part 311, and protects these optical members from making direct contact with the living tissue in the vagina. In addition, the holder 321 holds the irradiation part 323 and the light receiving part 324 so that the end surfaces of the irradiation part 323 and the light receiving part 324 do not protrude beyond the end surface of the ultrasound transmitting/receiving part 312.

For example, the irradiation part 323 may be a part with an optical system, such as a focus lens, provided at the end of the light guide fiber 322 for transmitting light, and emits light containing components of a specific wavelength from a range ranging from visible light to near-infrared light transmitted from the light source. The irradiation part 323 is attached to the holder 321 such that when the transvaginal probe 300 is inserted into the vagina, it can apply the light to the living tissue located in a predetermined direction.

For example, the light receiving part 324 may be a part with an optical system, such as a collector lens that collects light, provided at the end of the light guide fiber 325 for receiving light, and receives light containing components of a specific wavelength from a range ranging from visible light to near-infrared light. The light receiving part 324 is attached to the holder 321 such that when the transvaginal probe 300 is inserted into the vagina, it can receive reflected light, which is the light emitted from the irradiation part 323 and reflected from the living tissue located in the predetermined direction or transmitted light, which is transmitted through the living tissue.

The irradiation part 323 and the light receiving part 324 are provided at positions such that they are in a predetermined positional relationship with respect to the ultrasound transmitting/receiving part 312 when the holder 321 is placed over the insertion part 311. Specifically, the irradiation part 323 is provided such that it emits light in a direction parallel to the scanning plane of the ultrasound transmitted from the ultrasound transmitting/receiving unit 312, as shown in FIG. 20. In addition, the light receiving part 324 is provided such that it can receive reflected light or transmitted light that is emitted from the irradiation part 323 and is reflected by or transmitted through the living tissue to propagate in a direction parallel to the scanning plane. This allows for light application to the living tissue on the scanning plane, which is displayed as an ultrasound image, and reception of the reflected light or transmitted light. Here, "parallel to the ultrasound scanning plane" referred to herein refers, in addition to the condition where the light emitting direction (in other words, the orientation of the optical axis of the optical system provided on the irradiation part 323) or the light propagating direction (in other words, the orientation of the optical axis of the optical system provided on the light receiving part 324) is completely parallel to the scanning plane, to an approximately parallel range (e.g., the angle with respect to the scanning plane being within approximately +/- 5°) where the scanning plane does not intersect the emitting direction or propagating direction in the body of the subject.

In addition, if the ultrasound transmitting/receiving unit 312 employs the convex ultrasound scanning method, the irradiation part 323 may be provided such that it can emit light in a direction parallel to the ultrasound transmission direction at the center of the convex-shaped scanning plane. This allows for light application to the living tissue located at the center in the scanning direction of the ultrasound image.

As shown in FIG. 19, the irradiation part 323 and the light receiving part 324 are preferably arranged such that they are located opposite each other with the ultrasound transmitting/receiving part 312 sandwiched therebetween. In addition, the irradiation part 323 and the light receiving part 324 are more preferably arranged such that the line connecting the end surface of the irradiation part 323 and the end surface of the light receiving part 324 is orthogonal to the scanning direction of the ultrasound transmitted from the ultrasound transmitting/receiving part 312. Moreover, the irradiation part 323 and the light receiving part 324 are preferably arranged such that the line connecting the end surface of the irradiation part 323 and the end surface of the light receiving part 324 passes through substantially the center of the ultrasound scanning line. By arranging the irradiation part 323 and the light receiving part 324 in this manner, light can be emitted in a direction that is substantially parallel to the ultrasound scanning plane and that is substantially at the center of the ultrasound scanning range. This allows for a reliable light application to the ovarian cyst imaged in the ultrasound image, as shown in FIG. 20, and acquisition of a light signal containing information about the cyst fluid retained in the ovarian cyst.

From the viewpoint of applying light to the living tissue imaged in the ultrasound image and receiving the reflected or transmitted light, the irradiation part 323 and the light receiving part 324 are preferably arranged as close as possible to the ultrasound transmitting/receiving part 312. On the other hand, if the distance between the irradiation part 323 and the light receiving part 324 is too short, the scattered light, which is generated by the light emitted from the irradiation part 323 being scattered by the tissue in the living body, directly enters the light receiving part 324, and there is therefore a risk that the S/N ratio in the light signal received by the light receiving part 324 may decrease. In this regard, as shown in FIG. 19, by sandwiching the ultrasound transmitting/receiving part 312 between the irradiation part 323 and the light receiving part 324, the irradiation part 323 and the light receiving part 324 are allowed to be close to the ultrasound transmitting/receiving part 312 but a gap can be provided between the irradiation part 323 and the light receiving part 324, and the decrease in the S/N ratio in the light signal received by the light receiving part 324 can therefore be suppressed.

The center-to-center gap between the irradiation part 323 and the light receiving part 324 is preferably set roughly between 10 mm and 31 mm, inclusive. In order to suppress the direct entry of the scattered light of the light emitted from the irradiation part 323 into the light receiving part 324, the center-to-center distance is preferably set to 10 mm or more. To some extent, the longer the center-to-center distance is, the lower the noise caused by the scattered light may be. On the other hand, increasing the center-to-center distance increases the size of the tip portion of the transvaginal probe 300, and this makes it difficult to use the probe on the subjects. The center-to-center distance is therefore preferably be set to 31 mm or less in order to keep the outer dimensions compact. When an attachment 320 is used as in the present embodiment, the center-to-center distance is preferably set between 20 mm and 31 mm, inclusive, by taking into account the dimensions of the transvaginal probe 300.

As described above, according to the second embodiment of the present invention, by using a transvaginal probe in which an ultrasound transmitting/receiving part, a light irradiation part, and a light receiving part are provided in a predetermined positional relationship, the light emitting direction with respect to an ultrasound image can be understood. Therefore, a user can reliably apply light in the direction where the ovarian cyst is present, while observing the ultrasound image. Accordingly, the hemoglobin concentration in the cyst fluid retained in the ovarian cyst can be measured, in situ, in a non-invasive and stable manner based on the reflected light of the light applied in the above-described manner.

In addition, according to the second embodiment of the present invention, by attaching an attachment 320 to a general transvaginal ultrasound probe 310, a probe that is capable of measuring the hemoglobin concentration in the cystic fluid while referring to the ultrasound image can be realized in an inexpensive and convenient manner.

Example 4

(1) Creation of specimen (simulated cyst)

As with Example 1-1, hemoglobin aqueous solutions were prepared having six different concentrations: 0.0 g/dL (pure water only), 0.5 g/dL, 1.0 g/dL, 2.0 g/dL, 3.0 g/dL, and 4.0 g/dL. The prepared hemoglobin aqueous solutions were used as specimens to be tested, which are encapsulated in phantom models to be described later.

(2) Light application to simulated cysts and acquisition of absorbance

As described in the above-described second embodiment, a transvaginal ultrasound probe was prepared by attaching an attachment provided with a light irradiation part and a light receiving part to a transvaginal ultrasound probe (PVU-781 VTE) manufactured by former Toshiba Medical Systems Corporation (now Canon Medical Systems Corporation). The ultrasound probe was connected to an ultrasound diagnostic imaging apparatus (Xario100s). An optical fiber (type name: M29) manufactured by Thorlbas Inc. was used for the light guide fiber for transmitting light, and the same optical fiber was also used for the light guide fiber for receiving light. The center-to-center distance between the end surface of the irradiation part and the end surface of the receiving part was set to 30 mm. The same devices as those used in Example 1-1 were used for the light source (halogen lamp) and the spectrometer.

With respect to such transvaginal probe, ultrasound jelly (manufactured by JEX Co., Ltd.) was applied to the tip and a probe cover (manufactured by Fuji Latex Co., Ltd.) was placed thereover, as with the usual procedure for ultrasound diagnosis. The intention of following the usual procedure was to verify whether the light detection was still possible even if the light is attenuated by the ultrasonic jelly and the probe cover.

Phantom models of a female genital organ provided with an ovary mimicking an endometriotic ovarian cyst were prepared, and specimens to be tested were encapsulated therein. The phantom model was provided with an enlarged ovary, and optical tests and acquisition of ultrasound images could be performed simultaneously by encapsulating fluid inside the ovary. With respect to such phantom model, the light intensity $I_0$ measured by inserting the above-described transvaginal probe into the vagina and the light intensity $I_1$ measured, in a similar manner, with the specimen being injected into the ovary were acquired. Then, based on the light intensities $I_0$ and $I_1$, absorbance at each wavelength was calculated according to the Beer-Lambert law. Such measurement was performed six times (i.e., a total of 72 times) for the simulated cysts with six different concentrations and two different meat thicknesses. The ovary has an elliptical sphere shape with a long axis of approximately 50 mm and a short axis of approximately 30 mm. Signals were acquired by applying light to the ovary while checking the ultrasound image.

(3) Creation and verification of regression formula

Out of the 72 measurements (absorbance at 1025 points (wavelengths) per measurement) obtained by the above measurement, 15 measurements were randomly extracted and excluded, and the remaining 57 measurements were used for regression analysis to acquire a regression formula. As with Example 1-1, as for the regression analysis, PLS analysis was performed using the absorbance at all wavelengths at 1025 points as an explanatory variable, and the hemoglobin concentration obtained by actual measurement with the hematology analyzer as a response variable.

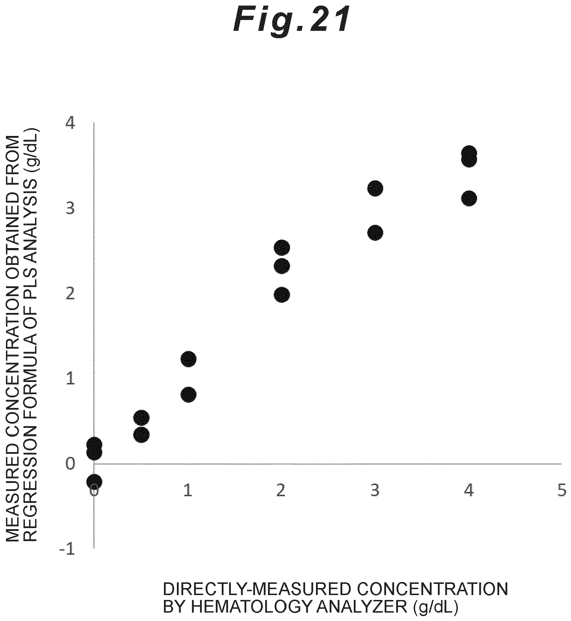

The hemoglobin concentration was calculated by substituting each of the 15 excluded measurements (same as above) into the regression formula obtained. Then, the correlation between the hemoglobin concentration calculated by the substitution into the regression formula and the hemoglobin concentration obtained by directly measuring the hemoglobin aqueous solution with the hematology analyzer was determined. FIG. 21 is a graph showing the correlation between the hemoglobin concentration measured directly by the hematology analyzer (directly-measured concentration) and the measured hemoglobin concentration calculated by the regression formula (calculated concentration). The correlation coefficient between the concentration measured directly by the hematology analyzer and the concentration calculated by the regression formula was R = 0.97, and it could be recognized that there was a good correlation between the two hemoglobin concentrations.

In Example 1-1 where the analysis using the above-described 1025 wavelengths were used, the center-to-center distance between the end surface of the irradiation part and the end surface of the receiving part was 18 mm, and the correlation coefficient R was 0.91. In contrast, in this Example 4, it can be considered that the correlation coefficient was improved because the noise scattered on the surface of the irradiation part was suppressed from being directly entering the light receiving part by increasing the center-to-center distance.

The present invention is not limited to the first and second embodiments described above, and may be carried out in various other forms within the scope of the invention as defined in the claims. For example, such various embodiments may be formed by excluding some components from all components shown in the above-described first and second embodiments, or by appropriately combining the components shown in the above-described first and second embodiments.

DESCRIPTION OF REFERENCE NUMBERS

**[0164]** 10 ... hemoglobin measuring system, 100 ... probe, 101, 323 ... irradiation part , 102, 324 ... light receiving part, 103, 312 ... ultrasound transmitting/receiving part, 110 ... main body, 111 ... light source, 112 ... spectroscopic part, 113 ... driving signal generation part, 114 ... ultrasound signal processing part, 115 ... operation input part, 116 ... display part, 120 ... storage part, 121 ... program storage part, 122 ... parameter storage part, 123 ... measurements storage part, 130 ... control part, 131 ... light source control part, 132 ... absorbance acquisition part, 133 ... concentration calculation part, 134 ... determination part, 135 ... scanning control part, 136 ... image processing part, 137 ... display control part, 200 ... simulated cyst, 201, 202, 212, 213 ... optical fibers, 203 ... container, 204 ... dark box, 210 ... probe, 211, 312 ... ultrasound transmitting/receiving part, 300 ... transvaginal probe, 310 ... ultrasound probe, 311 ... insertion part, 313 ... cable, 320 ... attachment, 321 ... holder, 322, 325 ... light guide fibers

**Claims**

1. A hemoglobin concentration measuring system (10), comprising:

   a transvaginal probe (100, 300), the transvaginal probe (100, 300) including:

   an ultrasound transmitting/receiving part (103, 312) that is capable of transmitting ultrasound to living tissue and capable of receiving an ultrasound echo reflected from the living tissue;
   a light irradiation part (101, 323) that is capable of emitting light in a direction parallel to a scanning plane of the ultrasound transmitted from the ultrasound transmitting/receiving part (103, 312), wherein the light contains components of a plurality of specific wavelengths from a wavelength region ranging from visible light to near-infrared light; and
   a light receiving part (102, 324) that is capable of receiving reflected light or transmitted light, wherein the reflected light or the transmitted light is light emitted from the light irradiation part (101, 323) and reflected by or transmitted through the living tissue to propagate in a direction parallel to the scanning plane;

   a display part (116) that is capable of displaying an ultrasound image containing an image of an ovarian cyst based on the ultrasound echo received by the ultrasound transmitting/receiving part (103, 312); and
   a concentration calculation part (133) that calculates hemoglobin concentration in a cystic fluid retained in the ovarian cyst based on an optical spectrum of the reflected light or the transmitted light from the ovarian cyst received by the light receiving part (102, 324),
   wherein the concentration calculation part (133) acquires absorbance measurement values at the plurality of specific wavelengths, and calculates the hemoglobin concentration by substituting the absorbance measurement values into a pre-acquired predetermined formula that represents the relationship between absorbance at the plurality of specific wavelengths and hemoglobin concentration,

wherein the components of the plurality of wavelengths include, at least, components of a wavelength in a visible light region, and

wherein the pre-acquired predetermined formula is a multiple regression formula constructed based on measurements using simulated cysts.

2. The hemoglobin concentration measuring system (10) according to claim 1, wherein the ultrasound transmitting/receiving part (312) transmits the ultrasound so as to scan the living tissue in a convex manner, and

the light irradiation part (323) is provided such that the light irradiation part (323) is capable of emitting the light in a direction parallel to an ultrasound transmission direction at the center of the convex-shaped scanning plane.

3. The hemoglobin concentration measuring system (10) according to claim 1 or 2, wherein the light irradiation part (323) and the light receiving part (102, 324) are arranged opposite each other with the ultrasound transmitting/receiving part (312) sandwiched therebetween.

4. The hemoglobin concentration measuring system (10) according to any one of claims 1 to 3, wherein the light irradiation part (323) and the light receiving part (324) are arranged such that a line connecting an end surface of the light irradiation part (323) and an end surface of the light receiving part (324) is orthogonal to a scanning direction of the ultrasound transmitted from the ultrasound transmitting/receiving part (312).

5. The hemoglobin concentration measuring system (10) according to any one of claims 1 to 4, wherein the center-to-center distance between the end surface of the light irradiation part (323) and the end surface of the light receiving part (324) is between 10 mm and 31 mm, inclusive.

6. The hemoglobin concentration measuring system (10) according to any one of claims 1 to 5, wherein the ultrasound transmitting/receiving part (312) is provided on a transvaginal ultrasound probe (310),

the light irradiation part (323) and the light receiving part (324) are attached to a holder (321) to be placed over an insertion part, which is to be inserted into the vagina, of the ultrasound probe, and
the light irradiation part (323) and the light receiving part (324), along with the holder (321), are detachable from the ultrasound probe.

7. The hemoglobin concentration measuring system (10) according to claim 1, wherein the components of the plurality of wavelengths are components of a wavelength in a visible light region.

8. The hemoglobin concentration measuring system (10) according to claim 7, wherein the plurality of specific wavelengths includes, at least, two or more wavelengths selected from: 580 nm, 590 nm, 640 nm, 680 nm, and 762 nm.

9. The hemoglobin concentration measuring system (10) according to any one of claims 1 to 8, further comprising a determination part (134) that determines under which classifications of degree of malignancy of an endometriotic ovarian cyst pre-associated with hemoglobin concentration the hemoglobin concentration calculated by the concentration calculation part (133) falls.

10. A hemoglobin concentration measuring method, comprising:

an ultrasound image displaying step (S 1) in which an ultrasound is transvaginally transmitted to living tissue, an ultrasound echo reflected from the living tissue is received, and an ultrasound image containing an image of an ovarian cyst is displayed based on the ultrasound echo;
a light irradiation step (S2) in which light is emitted in a direction parallel to a scanning plane of the ultrasound transmitted in the ultrasound image displaying step, wherein the light contains components of a plurality of specific wavelengths from a wavelength region ranging from visible light to near-infrared light;
a light receiving step (S3) in which reflected light or transmitted light is received, wherein the reflected light or the transmitted light is light emitted in the light irradiation step and reflected by or transmitted through the living tissue to propagate in a direction parallel to the scanning plane; and
a concentration calculation step (S4, S5) in which hemoglobin concentration in a cystic fluid retained in the ovarian cyst is calculated by a concentration calculation part (133) based on an optical spectrum of the reflected light or the transmitted light from the ovarian cyst received in the light receiving step (S3),

wherein the concentration calculation step (S4, S5) includes acquiring absorbance measurement values at the plurality of specific wavelengths, and calculating the hemoglobin concentration by substituting the absorbance measurement values into a pre-acquired predetermined formula that represents the relationship between absorbance at the plurality of specific wavelengths and hemoglobin concentration,

wherein the components of the plurality of wavelengths include, at least, components of a wavelength in a visible light region, and

wherein the pre-acquired predetermined formula is a multiple regression formula constructed based on measurements using simulated cysts.

**Patentansprüche**

1. Hämoglobinkonzentrationsmesssystem (10), umfassend:

   eine transvaginale Sonde (100, 300), wobei die transvaginale Sonde (100, 300) Folgendes einschließt:

      ein Ultraschallübertragungs-/-empfangsteil (103, 312), das dazu fähig ist, Ultraschall an Lebendgewebe zu übertragen, und dazu fähig ist, ein von dem Lebendgewebe reflektiertes Ultraschallecho zu empfangen;
      ein Lichtbestrahlungsteil (101, 323), das dazu fähig ist, Licht in einer Richtung parallel zu einer Abtastebene des aus dem Ultraschallübertragungs-/-empfangsteil (103, 312) übertragenen Ultraschalls zu emittieren, wobei das Licht Komponenten einer Vielzahl von spezifischen Wellenlängen aus einer Wellenlängenregion enthält, die von sichtbarem Licht bis Nahinfrarotlicht reicht; und
      ein Lichtempfangsteil (102, 324), das dazu fähig ist, reflektiertes Licht oder übertragenes Licht zu empfangen, wobei das reflektierte Licht oder das übertragene Licht aus dem Lichtbestrahlungsteil (101, 323) emittiertes und durch das Lebendgewebe reflektiertes oder durch dieses hindurch übertragenes Licht ist, um sich in einer Richtung parallel zu der Abtastebene auszubreiten;

   ein Anzeigeteil (116), das dazu fähig ist, ein Ultraschallbild, das ein Bild einer Ovarialzyste enthält, basierend auf dem durch das Ultraschallübertragungs-/-empfangsteil (103, 312) empfangenen Ultraschallecho anzuzeigen; und
   ein Konzentrationsberechnungsteil (133), das eine Hämoglobinkonzentration in einer in der Ovarialzyste zurückgehaltenen Zystenflüssigkeit basierend auf einem optischen Spektrum des reflektierten Lichts oder des übertragenen Lichts von der Ovarialzyste, das durch das Lichtempfangsteil (102, 324) empfangen wurde, berechnet,
   wobei das Konzentrationsberechnungsteil (133) Extinktionsmesswerte bei der Vielzahl von spezifischen Wellenlängen erfasst und die Hämoglobinkonzentration durch Einsetzen der Extinktionsmesswerte in eine vorab erfasste vorbestimmte Formel, die die Beziehung zwischen der Extinktion bei der Vielzahl von spezifischen Wellenlängen und der Hämoglobinkonzentration darstellt, berechnet,
   wobei die Komponenten der Vielzahl von Wellenlängen mindestens Komponenten einer Wellenlänge in einer Region des sichtbaren Lichts einschließen und
   wobei die vorab erfasste vorbestimmte Formel eine Formel der multiplen Regression ist, die basierend auf Messungen unter Verwendung simulierter Zysten aufgestellt wurde.

2. Hämoglobinkonzentrationsmesssystem (10) nach Anspruch 1, wobei das Ultraschallübertragungs-/-empfangsteil (312) den Ultraschall so überträgt, dass das Lebendgewebe auf konvexe Art und Weise abgetastet wird, und das Lichtbestrahlungsteil (323) derart bereitgestellt ist, dass das Lichtbestrahlungsteil (323) dazu fähig ist, das Licht in einer Richtung parallel zu einer Ultraschallübertragungsrichtung in der Mitte der konvex geformten Abtastebene zu emittieren.

3. Hämoglobinkonzentrationsmesssystem (10) nach Anspruch 1 oder 2, wobei das Lichtbestrahlungsteil (323) und das Lichtempfangsteil (102, 324) einander gegenüberliegend angeordnet sind, wobei das Ultraschallübertragungs-/-empfangsteil (312) dazwischen eingefügt ist.

4. Hämoglobinkonzentrationsmesssystem (10) nach einem der Ansprüche 1 bis 3, wobei das Lichtbestrahlungsteil (323) und das Lichtempfangsteil (324) derart angeordnet sind, dass eine Linie, die eine Endoberfläche des Lichtbestrahlungsteils (323) und eine Endoberfläche des Lichtempfangsteils (324) verbindet, orthogonal zu einer Abtastrichtung des aus dem Ultraschallübertragungs-/-empfangsteil (312) übertragenen Ultraschalls ist.

**5.** Hämoglobinkonzentrationsmesssystem (10) nach einem der Ansprüche 1 bis 4, wobei der Mittenabstand zwischen der Endoberfläche des Lichtbestrahlungsteils (323) und der Endoberfläche des Lichtempfangsteils (324) zwischen einschließlich 10 mm und 31 mm beträgt.

**6.** Hämoglobinkonzentrationsmesssystem (10) nach einem der Ansprüche 1 bis 5, wobei das Ultraschallübertragungs-/-empfangsteil (312) an einer transvaginalen Ultraschallsonde (310) bereitgestellt ist,

das Lichtbestrahlungsteil (323) und das Lichtempfangsteil (324) an einem Halter (321) angebracht sind, der über einem Einführteil, das in die Vagina einzuführen ist, der Ultraschallsonde zu platzieren ist, und

das Lichtbestrahlungsteil (323) und das Lichtempfangsteil (324) zusammen mit dem Halter (321) von der Ultraschallsonde abnehmbar sind.

**7.** Hämoglobinkonzentrationsmesssystem (10) nach Anspruch 1, wobei die Komponenten der Vielzahl von Wellenlängen Komponenten einer Wellenlänge in einer Region des sichtbaren Lichts sind.

**8.** Hämoglobinkonzentrationsmesssystem (10) nach Anspruch 7, wobei die Vielzahl von spezifischen Wellenlängen mindestens zwei oder mehr Wellenlängen einschließt, ausgewählt aus: 580 nm, 590 nm, 640 nm, 680 nm und 762 nm.

**9.** Hämoglobinkonzentrationsmesssystem (10) nach einem der Ansprüche 1 bis 8, ferner umfassend ein Bestimmungsteil (134), das bestimmt, unter welche Klassifikationen des Malignitätsgrads einer endometriotischen Ovarialzyste, die vorab mit der Hämoglobinkonzentration assoziiert wurden, die durch das Konzentrationsberechnungsteil (133) berechnete Hämoglobinkonzentration fällt.

**10.** Hämoglobinkonzentrationsmessverfahren, umfassend:

einen Ultraschallbild-Anzeigeschritt (S1), in dem ein Ultraschall transvaginal an Lebendgewebe übertragen wird, ein von dem Lebendgewebe reflektiertes Ultraschallecho empfangen wird und ein Ultraschallbild, das ein Bild einer Ovarialzyste enthält, basierend auf dem Ultraschallecho angezeigt wird;

einen Lichtbestrahlungsschritt (S2), in dem Licht in einer Richtung parallel zu einer Abtastebene des in dem Ultraschallbild-Anzeigeschritt übertragenen Ultraschalls emittiert wird, wobei das Licht Komponenten einer Vielzahl von spezifischen Wellenlängen aus einer Wellenlängenregion enthält, die von sichtbarem Licht bis Nahinfrarotlicht reicht;

einen Lichtempfangsschritt (S3), in dem reflektiertes Licht oder übertragenes Licht empfangen wird, wobei das reflektierte Licht oder das übertragene Licht in dem Lichtbestrahlungsschritt emittiertes und durch das Lebendgewebe reflektiertes oder durch dieses hindurch übertragenes Licht ist, um sich in einer Richtung parallel zu der Abtastebene auszubreiten; und

einen Konzentrationsberechnungsschritt (S4, S5), in dem eine Hämoglobinkonzentration in einer in der Ovarialzyste zurückgehaltenen Zystenflüssigkeit durch ein Konzentrationsberechnungsteil (133) basierend auf einem optischen Spektrum des reflektierten Lichts oder des übertragenen Lichts von der Ovarialzyste, das in dem Lichtempfangsschritt (S3) empfangen wurde, berechnet wird,

wobei der Konzentrationsberechnungsschritt (S4, S5) Erfassen von Extinktionsmesswerten bei der Vielzahl von spezifischen Wellenlängen und Berechnen der Hämoglobinkonzentration durch Einsetzen der Extinktionsmesswerte in eine vorab erfasste vorbestimmte Formel, die die Beziehung zwischen der Extinktion bei der Vielzahl von spezifischen Wellenlängen und der Hämoglobinkonzentration darstellt, einschließt,

wobei die Komponenten der Vielzahl von Wellenlängen mindestens Komponenten einer Wellenlänge in einer Region des sichtbaren Lichts einschließen und

wobei die vorab erfasste vorbestimmte Formel eine Formel der multiplen Regression ist, die basierend auf Messungen unter Verwendung simulierter Zysten aufgestellt wurde.

**Revendications**

**1.** Système de mesure de concentration d'hémoglobine (10), comprenant :

une sonde transvaginale (100, 300), la sonde transvaginale (100, 300) comprenant :

une partie de transmission/réception d'ultrasons (103, 312) qui est capable de transmettre des ultrasons à un tissu vivant et capable de recevoir un écho ultrasonore réfléchi par le tissu vivant ;

une partie d'irradiation de lumière (101, 323) qui est capable d'émettre de la lumière suivant une direction parallèle à un plan de balayage des ultrasons transmis par la partie d'émission/réception d'ultrasons (103, 312), ladite lumière contenant des composantes d'une pluralité de longueurs d'onde spécifiques à partir d'une zone de longueurs d'onde allant de la lumière visible à la lumière proche infrarouge ; et

une partie de réception de lumière (102, 324) qui est capable de recevoir une lumière réfléchie ou une lumière transmise, ladite lumière réfléchie ou ladite lumière transmise étant une lumière émise par la partie d'irradiation de lumière (101, 323) et réfléchie par le tissu vivant ou transmise à travers celui-ci de manière à se propager suivant une direction parallèle au plan de balayage ;

une partie d'affichage (116) qui est capable d'afficher une image ultrasonore contenant l'image d'un kyste ovarien sur la base de l'écho ultrasonore reçu par la partie d'émission/réception d'ultrasons (103, 312) ; et

une partie de calcul de concentration (133) qui calcule la concentration d'hémoglobine dans un liquide kystique retenu dans le kyste ovarien sur la base d'un spectre optique de la lumière réfléchie ou de la lumière transmise par le kyste ovarien reçue par la partie de réception de lumière (102, 324),

ladite partie de calcul de concentration (133) acquérant des valeurs de mesure d'absorbance à la pluralité de longueurs d'onde spécifiques, et calculant la concentration d'hémoglobine en substituant les valeurs de mesure d'absorbance dans une formule prédéfinie pré-acquise qui représente la relation entre l'absorbance à la pluralité de longueurs d'onde spécifiques et la concentration d'hémoglobine,

lesdites composantes de la pluralité de longueurs d'onde comprenant, au moins, des composantes d'une longueur d'onde dans une zone de lumière visible, et

ladite formule prédéfinie pré-acquise étant une formule de régression multiple construite à partir de mesures en utilisant des kystes simulés.

2. Système de mesure de concentration d'hémoglobine (10) selon la revendication 1, ladite partie d'émission/réception d'ultrasons (312) transmettant les ultrasons de manière à balayer le tissu vivant de manière convexe, et

ladite partie d'irradiation de lumière (323) étant prévue de sorte que la partie d'irradiation de lumière (323) soit capable d'émettre la lumière suivant une direction parallèle à une direction de transmission d'ultrasons au centre du plan de balayage de forme convexe.

3. Système de mesure de concentration d'hémoglobine (10) selon la revendication 1 ou 2, ladite partie d'irradiation de lumière (323) et ladite partie de réception de lumière (102, 324) étant disposées l'une en face de l'autre, la partie d'émission/réception d'ultrasons (312) étant prise en sandwich entre elles.

4. Système de mesure de concentration d'hémoglobine (10) selon l'une quelconque des revendications 1 à 3, ladite partie d'irradiation de lumière (323) et ladite partie de réception de lumière (324) étant disposées de sorte qu'une ligne reliant une surface d'extrémité de la partie d'irradiation de lumière (323) et une surface d'extrémité de la partie de réception de lumière (324) soit orthogonale à la direction de balayage des ultrasons transmis par la partie d'émission/réception d'ultrasons (312).

5. Système de mesure de concentration d'hémoglobine (10) selon l'une quelconque des revendications 1 à 4, ladite distance centre à centre entre la surface d'extrémité de la partie d'irradiation de lumière (323) et la surface d'extrémité de la partie de réception de lumière (324) étant comprise entre 10 mm et 31 mm, inclus.

6. Système de mesure de concentration d'hémoglobine (10) selon l'une quelconque des revendications 1 à 5, ladite partie d'émission/réception d'ultrasons (312) étant disposée sur une sonde à ultrasons transvaginale (310),

ladite partie d'irradiation de lumière (323) et ladite partie de réception de lumière (324) étant fixées à un support (321) destiné à être placé sur une partie d'insertion, qui doit être insérée dans le vagin, de la sonde à ultrasons, et ladite partie d'irradiation de lumière (323) et ladite partie de réception de lumière (324), ainsi que ledit support (321), étant détachables de la sonde à ultrasons.

7. Système de mesure de concentration d'hémoglobine (10) selon la revendication 1, lesdites composantes de la pluralité de longueurs d'onde étant des composantes d'une longueur d'onde dans une zone de lumière visible.

8. Système de mesure de concentration d'hémoglobine (10) selon la revendication 7, ladite pluralité de longueurs d'onde spécifiques comprenant au moins deux longueurs d'onde ou plus choisies parmi : 580 nm, 590 nm, 640 nm, 680 nm et 762 nm.

**9.** Système de mesure de concentration d'hémoglobine (10) selon l'une quelconque des revendications 1 à 8, comprenant en outre une partie de détermination (134) qui détermine sous quelles classifications de degré de malignité d'un kyste ovarien endométriosique pré-associé à la concentration d'hémoglobine tombe la concentration d'hémoglobine calculée par la partie de calcul de concentration (133).

**10.** Procédé de mesure de concentration d'hémoglobine, comprenant :

une étape d'affichage d'image ultrasonore (S1) dans laquelle des ultrasons sont transmis par voie transvaginale à un tissu vivant, un écho ultrasonore réfléchi par le tissu vivant est reçu, et une image ultrasonore contenant une image d'un kyste ovarien est affichée sur la base de l'écho ultrasonore ;
une étape d'irradiation de lumière (S2) dans laquelle la lumière est émise suivant une direction parallèle à un plan de balayage des ultrasons transmis dans l'étape d'affichage d'image ultrasonore, ladite lumière contenant des composantes d'une pluralité de longueurs d'onde spécifiques à partir d'une zone de longueurs d'onde allant de la lumière visible à la lumière proche infrarouge ;
une étape de réception de lumière (S3) dans laquelle une lumière réfléchie ou une lumière transmise est reçue, ladite lumière réfléchie ou ladite lumière transmise étant une lumière émise dans l'étape d'irradiation de lumière et réfléchie par le tissu vivant ou transmise à travers celui-ci de manière à se propager suivant une direction parallèle au plan de balayage ; et
une étape de calcul de concentration (S4, S5) dans laquelle la concentration d'hémoglobine dans un liquide kystique retenu dans le kyste ovarien est calculée par une partie de calcul de concentration (133) sur la base d'un spectre optique de la lumière réfléchie ou de la lumière transmise par le kyste ovarien reçue dans l'étape de réception de lumière (S3),
ladite étape de calcul de concentration (S4, S5) comprenant l'acquisition de valeurs de mesure d'absorbance à la pluralité de longueurs d'onde spécifiques, et le calcul de la concentration d'hémoglobine en substituant les valeurs de mesure d'absorbance dans une formule prédéfinie pré-acquise qui représente la relation entre l'absorbance à la pluralité de longueurs d'onde spécifiques et la concentration d'hémoglobine,
lesdites composantes de la pluralité de longueurs d'onde comprenant, au moins, des composantes d'une longueur d'onde dans une zone de lumière visible, et
ladite formule prédéfinie pré-acquise étant une formule de régression multiple construite sur la base de mesures en utilisant des kystes simulés.

# *Fig.1*

| CASE NO. | POSTOPERATIVE DEFINITE DIAGNOSES | HEMOGLOBIN CONCENTRATION (g/dL) |
|---|---|---|
| 1 | LEFT OVARIAN CANCER | 0.72 |
| | RIGHT ENDOMETRIOTIC CYST | 13.37 |
| 2 | LEFT OVARIAN TUMOR | 8.12 |
| | RIGHT OVARIAN TUMOR | 5.61 |
| 3 | LEFT OVARIAN TUMOR | 3.94 |
| 4 | LEFT OVARIAN TUMOR | 17.31 |
| 5 | RIGHT ENDOMETRIOTIC CYST | 3.55 |
| | LEFT ENDOMETRIOTIC CYST | 6.80 |
| 6 | LEFT ENDOMETRIOTIC CYST | 2.68 |
| | RIGHT ENDOMETRIOTIC CYST | 11.53 |
| 7 | LEFT ENDOMETRIOTIC CYST | 10.72 |
| 8 | RIGHT ENDOMETRIOTIC CYST | 8.73 |
| 9 | LEFT ENDOMETRIOTIC CYST | 2.65 |
| 10 | RIGHT ENDOMETRIOTIC CYST | 21.72 |
| 11 | LEFT ENDOMETRIOTIC CYST | 8.71 |
| 12 | CLEAR CELL ADENOCARCINOMA | 1.79 |
| 13 | ENDOMETRIOTIC CYST | 15.29 |
| 14 | ENDOMETRIOTIC CYST | 16.14 |
| 15 | LEFT OVARIAN CANCER | 0.47 |
| 16 | LEFT ENDOMETRIOTIC CYST | 18.78 |
| 17 | RIGHT OVARIAN TUMOR | 0.58 |
| 18 | RIGHT ENDOMETRIOTIC CYST | 4.82 |
| 19 | RIGHT ENDOMETRIOTIC CYST | 6.01 |
| 20 | LEFT ENDOMETRIOTIC CYST | 5.16 |

# Fig.2

# Fig.3

```
                    ┌──────────┐
                    │  START   │
                    └──────────┘
                          │
                          ▼
        ┌─────────────────────────────────────┐
        │      DISPLAY ULTRASOUND IMAGE        │──── S1
        └─────────────────────────────────────┘
                          │
                          ▼
        ┌─────────────────────────────────────┐
        │      APPLY LIGHT TO LIVING TISSUE    │──── S2
        └─────────────────────────────────────┘
                          │
                          ▼
        ┌─────────────────────────────────────┐
        │         RECEIVE REFLECTED OR         │
        │ TRANSMITTED LIGHT FROM LIVING TISSUE │──── S3
        └─────────────────────────────────────┘
                          │
                          ▼
        ┌─────────────────────────────────────┐
        │         ACQUIRE ABSORBANCE           │
        │    MEASUREMENTS AT A PLURALITY OF    │──── S4
        │        SPECIFIC WAVELENGTHS          │
        └─────────────────────────────────────┘
                          │
                          ▼
        ┌─────────────────────────────────────┐
        │ CALCULATE BIOMARKER CONCENTRATION    │
        │  BY SUBSTITUTING ABSORBANCE          │
        │ MEASUREMENTS INTO PREDETERMINED      │──── S5
        │            FORMULA                   │
        └─────────────────────────────────────┘
                          │
                          ▼
                    ┌──────────┐
                    │   END    │
                    └──────────┘
```

# Fig.4

```
                    ┌─────────────┐
                    │    START    │
                    └──────┬──────┘
                           │
                           ▼
              ╱───────────────────────╲
              │        LOOP A          │
              ╲───────────────────────╱
                           │
                           ▼
        ┌───────────────────────────────────┐
        │ APPLY LIGHT TO SIMULATED LIVING BODY│  ── S11
        │             SAMPLE                  │
        └──────────────────┬──────────────────┘
                           │
                           ▼
        ┌───────────────────────────────────┐
        │  RECEIVE REFLECTED OR TRANSMITTED  │
        │  LIGHT FROM SIMULATED LIVING BODY  │  ── S12
        │             SAMPLE                  │
        └──────────────────┬──────────────────┘
                           │
                           ▼
        ┌───────────────────────────────────┐
        │  ACQUIRE OPTICAL SPECTRUM OF        │  ── S13
        │       RECEIVED LIGHT                │
        └──────────────────┬──────────────────┘
                           │
                           ▼
              ╱───────────────────────╲
              │        LOOP A          │
              │   REPEAT FOR A SET     │
              │   NUMBER OF TIMES      │
              ╲───────────────────────╱
                           │
                           ▼
        ┌───────────────────────────────────┐
        │ EXTRACT A PLURALITY OF WAVELENGTHS │  ── S14
        │ BASED ON A PLURALITY OF OPTICAL    │
        │             SPECTRA                 │
        └──────────────────┬──────────────────┘
                           │
                           ▼
        ┌───────────────────────────────────────┐
        │ DETERMINE FORMULA REPRESENTING THE     │
        │ RELATIONSHIP BETWEEN ABSORBANCE AT     │  ── S15
        │ THE PLURALITY OF WAVELENGTHS AND       │
        │ BIOMARKER CONCENTRATION                │
        └──────────────────┬──────────────────┘
                           │
                           ▼
                    ┌─────────────┐
                    │     END     │
                    └─────────────┘
```

EP 4 137 063 B1

# *Fig.5*

<u>10</u>

# Fig.6

TO LIGHT SOURCE

TO SPECTROMETER

204

200

203

201

202

18mm

## Fig.7

## Fig.8

## Fig.9

MEASURED CONCENTRATION OBTAINED FROM REGRESSION FORMULA OF PLS ANALYSIS (g/dL)

DIRECTLY-MEASURED CONCENTRATION BY HEMATOLOGY ANALYZER (g/dL)

## Fig.10

MEASURED CONCENTRATION OBTAINED FROM REGRESSION FORMULA OF PLS ANALYSIS (g/dL)

DIRECTLY-MEASURED CONCENTRATION BY HEMATOLOGY ANALYZER (g/dL)

# Fig.11

| USED WAVELENGTHS (nm) | R |
|---|---|
| 580 680 | 0.86 |
| 700 800 | 0.83 |
| 900 968 | 0.89 |
| 932 958 | 0.85 |
| 640 932 958 | 0.89 |
| 580 680 978 | 0.84 |
| 762 900 958 | 0.87 |
| 762 900 958 978 | 0.87 |
| 580 590 640 680 | 0.86 |
| 580 590 640 680 762 876 | 0.88 |
| 900 932 958 968 978 1095 | 0.88 |
| 580 640 762 900 958 978 | 0.88 |
| 590 680 876 932 968 1095 | 0.85 |

# Fig.12

# Fig.13

# Fig.14

# Fig.15

# Fig.16

# Fig.17

# Fig.18

# *Fig.19*

300

312

311

321

SCANNING
DIRECTION

323

324

# *Fig.20*

ULTRASOUND IMAGE
(SCANNING PLANE)

ULTRASOUND
TRANSMISSION
DIRECTION

SCANNING
DIRECTION

LIGHT EMITTING
DIRECTION

## Fig.21

MEASURED CONCENTRATION OBTAINED FROM REGRESSION FORMULA OF PLS ANALYSIS (g/dL)

DIRECTLY-MEASURED CONCENTRATION BY HEMATOLOGY ANALYZER (g/dL)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018163174 A
- JP 2002122537 A
- JP 6657438 B
- EP 3124977 A1

### Non-patent literature cited in the description

- **C. YOSHIMOTO.** Cyst fluid iron-related compounds as useful markers to distinguish malignant transformation from benign endometriotic cysts. *Cancer Biomarkers,* 2015, vol. 15, 493-499
- Development of Optical Transvaginal Probe that can Evaluate the Malignant transformation of Endometriosis in a Minimally Invasive Manner. *Report on Research and Development Result- Strategic Fundamental Technology Advancement Support Project - Strategic Fundamental Technology Advancement/Cooperation Support Project in FY2017,* October 2018, https://www.chusho.meti.go.jp/keiei/sapoin/portal/seika/28fy.htm
- **C. YOSHIMOTO et al.** Cyst fluid iron-related compounds as useful markers to distinguish malignant transformation from benign endometriotic cysts. *Cancer Biomarkers,* 2015, vol. 15, 493-499